# EUROPEAN PATENT APPLICATION

(11) **EP 2 605 013 A1**
(43) Date of publication of application: **19.06.2013**
(21) Application number: 12191514.4
(22) Date of filing: 07.06.2006
(51) Int. Cl.: G01N 33/53, C12P 21/06, C12N 15/00, C07K 1/00, C07K 14/00, C07K 17/00, C07H 21/04, A61K 38/16

(54) **Stimulation of pancreatic beta-cell proliferation**

(30) Priority: 07.06.2005 US 687856 P; 05.12.2005 US 741893 P
(62) Divisional of application: 06772490.6
(71) Applicant: THE ROCKEFELLER UNIVERSITY, New York, NY 10021 (US)
(72) Inventor: Stoffel, Markus, 8044 Zurich (CH); Akpinar, Pinar, Santa Monica, CA 90401 (US)
(74) Representative: Thurgood, Alexander John

(57) **Abstract**

This invention provides a polypeptide, TMEM27, and secreted forms thereof, nucleic acids and constructs encoding the same, and cells comprising the same. The TMEM27 protein is expressed in hormone positive cells at early stages of pancreas development and pancreatic beta-cells in the mature pancreas, whose expression promotes pancreatic beta-cell replication and increased islet mass. Applications of the protein in diagnostics and therapeutics are described.

SEQUENCE LISTING

<110>  STOFFEL, Markus
       AKPINAR, Pinar

<120>  STIMULATION OF PANCREATIC B CELL PROLIFERATION

<130>  P-7925-PC

<140>  Not Yet Known
<141>  2006-06-04

<160>  19

<170>  PatentIn version 3.3

<210>  1
<211>  19
<212>  PRT
<213>  Mus musculus

<400>  1

Val Gln Ser Ala Ile Arg Lys Asn Arg Asn Arg Ile Asn Ser Ala Phe
1               5                   10                  15

Phe Leu Asp

<210>  2
<211>  20
<212>  PRT
<213>  Mus musculus

<400>  2

Gly Ile Pro Cys Asp Pro Leu Asp Met Lys Gly Gly His Ile Asn Asp
1               5                   10                  15

Gly Phe Leu Thr
            20

<210>  3
<211>  24
<212>  DNA
<213>  Mus musculus

<400>  3
ggagattttc gtaaataact gaca                                              24

<210>  4
<211>  24
<212>  DNA
<213>  Mus musculus

<400>  4
gggcgttaat tattaaacct ttta                                              24

<210>  5
<211>  24
<212>  DNA
<213>  Mus musculus

<400>  5
gggcagagat tattaaacct ttta                                              24


<210>  6
<211>  24
<212>  DNA
<213>  Mus musculus

<400>  6
acaggaggca ggtgggaggc ttct                                              24


<210>  7
<211>  22
<212>  DNA
<213>  Mus musculus

<400>  7
cccggattag ggtatcggag aa                                                22


<210>  8
<211>  19
<212>  DNA
<213>  Mus musculus

<400>  8
gggctcagct ttcctccta                                                    19


<210>  9
<211>  24
<212>  DNA
<213>  Mus musculus

<400>  9
ctccgcctta actgttctct gatg                                              24


<210>  10
<211>  131
<212>  DNA
<213>  Homo sapiens

<400>  10
gaaaacaact aatctgtttt ccaaacaggt tttcataaat aactgacagg agatgggctg      60

tttttatgga agtattaacc caaatgatgg gtgttaatca ttaaaccttt ttggtggtgg     120

ctcgcttgtt t                                                           131


<210>  11
<211>  133
<212>  DNA
<213>  Mus musculus

<400>  11
gaagaaccca ctaacctgct tctcaaatag attttcgtaa ataactgaca ggggatggtt      60

cgcttctatg gacgtattaa cccaactgat gggcgttaat tattaaacct tttagatggt     120

ggctcgctga ttt                                                         133


<210>  12

<211>  15
<212>  DNA
<213>  Homo sapiens

<400>  12
cataaataac tgaca                                                        15


<210>  13
<211>  16
<212>  DNA
<213>  Homo sapiens

<400>  13
ggtgttaatc attaaa                                                       16


<210>  14
<211>  15
<212>  DNA
<213>  Mus musculus

<400>  14
cgtaaataac tgaca                                                        15


<210>  15
<211>  16
<212>  DNA
<213>  Mus musculus

<400>  15
ggcgttaatt attaaa                                                       16


<210>  16
<211>  222
<212>  PRT
<213>  Mus musculus

<400>  16

Met Leu Trp Leu Leu Phe Phe Leu Val Thr Ala Ile His Ala Glu Leu
1               5                   10                  15


Cys Gln Pro Gly Ala Glu Asn Ala Phe Lys Val Arg Leu Ser Ile Arg
            20                  25                  30


Thr Ala Leu Gly Asp Lys Ala Tyr Ala Trp Asp Thr Asn Glu Glu Tyr
        35                  40                  45


Leu Phe Lys Ala Met Val Ala Phe Ser Met Arg Lys Val Pro Asn Arg
    50                  55                  60


Glu Ala Thr Glu Ile Ser His Val Leu Leu Cys Asn Val Thr Gln Arg
65                  70                  75                  80


Val Ser Phe Trp Phe Val Val Thr Asp Pro Ser Lys Asn His Thr Leu
                85                  90                  95


Pro Ala Val Glu Val Gln Ser Ala Ile Arg Met Asn Lys Asn Arg Ile
            100                 105                 110

Asn Asn Ala Phe Phe Leu Asn Asp Gln Thr Leu Glu Phe Leu Lys Ile
        115                 120                 125

Pro Ser Thr Leu Ala Pro Pro Met Asp Pro Ser Val Pro Ile Trp Ile
    130                 135                 140

Ile Ile Phe Gly Val Ile Phe Cys Ile Ile Ile Val Ala Ile Ala Leu
145                 150                 155                 160

Leu Ile Leu Ser Gly Ile Trp Gln Arg Arg Arg Lys Asn Lys Glu Pro
                165                 170                 175

Ser Glu Val Asp Asp Ala Glu Asp Lys Cys Glu Asn Met Ile Thr Ile
            180                 185                 190

Glu Asn Gly Ile Pro Ser Asp Pro Leu Asp Met Lys Gly Gly His Ile
        195                 200                 205

Asn Asp Ala Phe Met Thr Glu Asp Glu Arg Leu Thr Pro Leu
    210                 215                 220

<210>  17
<211>  815
<212>  DNA
<213>  Mus musculus

<400>  17
atgtcattgc acccaatgac tttaacgctc agtccttgga taccccacat gtgagacggt      60

tgtaaccact gtaaggattt gagaaataat cagggagcct agctcacagt aagtagttct     120

tagtcattgt taaatagttg cgctgaacaa tgttgtcact agcactgtgt agcaacttaa     180

ctaaatgaca ggttcttagc agttctagcc ccttttaaag ggttagcatt catttcttcc     240

tagtggcacc aggacagagc actgttcagg gaaggtatca ccatcaccat caccatcacc     300

atcgtcatca ccatcaccat cgtcatcacc caatgatcat catctccagc gtgaggcttt     360

atatcattaa tgttgagttt tagacagtag ccagatctta gagtttaacc tagccatcct     420

ctagttggga attgttaacc aaccaacagt ctaggaagtc ttgttccctt tactctccag     480

aggtgaaaaa ggagttatat ttgtttccta agtatggtag cctgtctttg aatcagattg     540

gttagcctga aagggcacaa agattatgcc aaagaaaact ccatgctctc cacgtttagc     600

ttaaccaaac aactacagga ggcaggtggg aggcttctga ttgtgtcagc tcaggaaaca     660

ttcttatgag gtagagtttg aggaagaacc cactaacctg cttctcaaat agattttcgt     720

aaataactga caggggatgg ttcgcttcta tggacgtatt aacccaactg atgggcgtta     780

attattaaac cttttagatg gtggctcgct gattt                                815

<210>  18
<211>  815
<212>  DNA

Page 4

<213>  Mus musculus

<400>  18
atgtcattgc acccaatgac tttaacgctc agtccttgga taccccacat gtgagacggt      60

tgtaaccact gtaaggattt gagaaataat cagggagcct agctcacagt aagtagttct     120

tagtcattgt taaatagttg cgctgaacaa tgttgtcact agcactgtgt agcaacttaa     180

ctaaatgaca ggttcttagc agttctagcc ccttttaaag ggttagcatt catttcttcc     240

tagtggcacc aggacagagc actgttcagg gaaggtatca ccatcaccat caccatcacc     300

atcgtcatca ccatcaccat cgtcatcacc caatgatcat catctccagc gtgaggcttt     360

atatcattaa tgttgagttt tagacagtag ccagatctta gagtttaacc tagccatcct     420

ctagttggga attgttaacc aaccaacagt ctaggaagtc ttgttccctt tactctccag     480

aggtgaaaaa ggagttatat ttgtttccta agtatggtag cctgtctttg aatcagattg     540

gttagcctga aagggcacaa agattatgcc aaagaaaact ccatgctctc cacgtttagc     600

ttaaccaaac aactacagga ggcaggtggg aggcttctga ttgtgtcagc tcaggaaaca     660

ttcttatgag gtagagtttg aggaagaacc cactaacctg cttctcaaat agattttcgt     720

aaataacact gaggggatgg ttcgcttcta tggacgtatt aacccaactg atgggcgtta     780

attattaaac cttttagatg gtggctcgct gattt                                815

<210>  19
<211>  815
<212>  DNA
<213>  Mus musculus

<400>  19
atgtcattgc acccaatgac tttaacgctc agtccttgga taccccacat gtgagacggt      60

tgtaaccact gtaaggattt gagaaataat cagggagcct agctcacagt aagtagttct     120

tagtcattgt taaatagttg cgctgaacaa tgttgtcact agcactgtgt agcaacttaa     180

ctaaatgaca ggttcttagc agttctagcc ccttttaaag ggttagcatt catttcttcc     240

tagtggcacc aggacagagc actgttcagg gaaggtatca ccatcaccat caccatcacc     300

atcgtcatca ccatcaccat cgtcatcacc caatgatcat catctccagc gtgaggcttt     360

atatcattaa tgttgagttt tagacagtag ccagatctta gagtttaacc tagccatcct     420

ctagttggga attgttaacc aaccaacagt ctaggaagtc ttgttccctt tactctccag     480

aggtgaaaaa ggagttatat ttgtttccta agtatggtag cctgtctttg aatcagattg     540

gttagcctga aagggcacaa agattatgcc aaagaaaact ccatgctctc cacgtttagc     600

ttaaccaaac aactacagga ggcaggtggg aggcttctga ttgtgtcagc tcaggaaaca     660

ttcttatgag gtagagtttg aggaagaacc cactaacctg cttctcaaat agattttcgt     720

aaataactga caggggatgg ttcgcttcta tggacgtatt aacccaactg atgggccaat     780

attattaaac cttttagatg gtggctcgct gattt                                815

## Description

### FIELD OF THE INVENTION

This invention provides a novel protein involved in pancreatic β-cell proliferation and/or insulin secretion, nucleic acids and constructs encoding the same, and cells comprising the same. The invention also provides diagnostic and therapeutic applications of the TMEM27 protein.

### BACKGROUND OF THE INVENTION

Pancreatic β-cell hyperplasia is an important adaptive mechanism to maintain normoglycemia during physiological growth and in obesity. Increasing evidence suggests that the β-cell mass is dynamic and that increased demands on insulin secretion in insulin resistance and pregnancy can lead to rapid and marked changes in the β-cell mass. The mass of β-cells is governed by the balance of β-cell growth (replication) and by β-cell death (apoptosis), however, the molecular basis of the factors that control β-cell mass remain elusive. Understanding how β-cell mass is regulated is important to design rational approaches to prevent pancreatic β-cell loss in insulin resistant states and to expand β-cells for transplantation in type 1 diabetes.

During development, β-cells are generated from a population of pancreatic progenitor cells. The β-cells that differentiate from progenitor cells are postmitotic, and direct lineage tracing studies indicate that a population of progenitor cells persists throughout embryogenesis to allow the differentiation of new β-cells. In the neonatal period new β-cells are formed by replication of differentiated β-cells, which results in a massive increase in β-cell mass. In adulthood there is little increase in the β-cell number except in conditions of increased demand. During pregnancy a marked hyperplasia of the β-cells is observed both in rodents and man. This is due to increased mitotic activity of β-cells exposed to placental lactogen (PL), prolactin (PRL) and growth hormone (GH). The growth stimulatory signals in pathological insulin resistant states are less well understood.

Several mouse models of insulin resistance and diabetes, such as the *ob*/*ob* and *db*/*db* mice or mutant mice created by inactivation of the gene for insulin receptor substrate-1 (IRS-1) or double heterozygous (DH) knockout of the insulin receptor and IRS-1, exhibit marked islet hyperplasia. In contrast, loss of IRS2 function leads to a dramatic reduction of β-cells and diabetes. Glucose itself is known to stimulate β-cell replication, however, many of the above mouse models increase their total islet mass before the onset of detectable hyperglycemia. Furthermore, in most cases the hyperplastic response bears no relationship to the level of hyperglycemia, suggesting that factors independent of glucose likely contribute to the islet growth.

Endocrine pancreatic growth during development depends on multiple transcription factors that display highly specific temporal and spatial expression patterns that control mechanisms for cell differentiation. During early pancreatic development, cell fate decisions and differentiation of endocrine progenitor cells into hormone-producing islet cells is tightly regulated by the sequential expression of specific transcription factors. The expression of these factors is also important for maintaining normal pancreatic β-cell function in adult life. For instance, mutations in several transcription factors lead to a subtype of type 2 diabetes called maturity-onset diabetes of the young (MODY), which are characterized by autosomal dominant inheritance, an early age of disease-onset, and development of marked hyperglycemia with a progressive impairment in insulin secretion. The most frequent form of MODY is caused by mutations in the gene encoding hepatocyte nuclear factor (Tcf1). Mutant mice with loss of Tcf1 function as well as transgenic mice expressing a naturally occurring dominant-negative form of human HNF-1 (P291fsinsC) in pancreatic β-cells exhibit progressive hyperglycemia with age due to impaired glucose-stimulated insulin secretion. These mice exhibit a progressive reduction in β-cell number, proliferation rate, and pancreatic insulin content. These data suggest that Tcf-1 target genes are also required for maintenance of normal β-cell mass, though their identity is as yet unknown.

### SUMMARY OF THE INVENTION

In one embodiment, this invention provides an isolated nucleic acid comprising a regulatory region of a gene encoding a TMEM27 protein, wherein said regulatory region comprises at least one high-affinity binding site for a Tcf1 protein. In one embodiment, the regulatory region has a nucleic acid sequence corresponding to or homologous to SEQ ID NO: 10 or 11. In another embodiment, the binding site comprises a nucleic acid sequence corresponding to or homologous to SEQ ID NO: 12, 13, 14, or 15. In another embodiment, this invention provides a cell, or in another embodiment, a vector comprising a nucleic acid of this invention.

In another embodiment, this invention provides an isolated polypeptide, comprising a secreted form of a TMEM27 protein, wherein said polypeptide is about 25 kDa in size, and comprises an N-terminal fragment of a sequence corresponding to, or homologous to, SEQ ID NO: 16.

In another embodiment, this invention provides a method of increasing pancreatic β-cell mass, said method comprising contacting a pancreatic β-cell or a cell which may be induced to become a pancreatic β-cell with a TMEM27 polypeptide and providing conditions favorable for β-cell proliferation.

In another embodiment, this invention provides a method of increasing pancreatic β-cell mass, said method comprising contacting a pancreatic β-cell or a cell which may be induced to become a pancreatic β-cell with a nucleic acid encoding a TMEM27 polypeptide and providing conditions favorable for expression of said nucleic acid.

In another embodiment, this invention provides a method of altering metabolism in a subject, said method comprising contacting a pancreatic β-cell or a cell which may be induced to become a pancreatic β-cell with a TMEM27 polypeptide or a nucleic acid encoding said TMEM27 polypeptide.

In another embodiment, this invention provides a method of inhibiting, suppressing or treating diabetes in a subject, said method comprising contacting a pancreatic β**-**cell or a cell which may be induced to become a pancreatic β-cell with a TMEM27 polypeptide or a nucleic acid encoding said TMEM27 polypeptide.

In another embodiment, this invention provides a method of assessing pancreatic islet mass in a subject, the method comprising the step of determining a concentration of TMEM27 in the serum of said subject and comparing said concentration versus that of a control.

In another embodiment, this invention provides a method for identifying proteins which interact with a TMEM27 protein, or a secreted form thereof, the method comprising the step of contacting a TMEM27 polypeptide with a molecule of interest for a time and under conditions sufficient to facilitate specific interaction between said polypeptide and said molecule of interest and identifying said molecule of interest.

In another embodiment, this invention provides a method of increasing pancreatic β-cell mass, said method comprising contacting a pancreatic β-cell or a cell which may be induced to become a pancreatic β- with a serine or metallo protease inhibitor and providing conditions favorable for expression of said nucleic acid.

In another embodiment, this invention provides a method of altering metabolism in a subject, said method comprising contacting a pancreatic β-cell or a cell which may be induced to become a pancreatic β-cell with a serine or metallo protease inhibitor.

In another embodiment, this invention provides a method of inhibiting, suppressing or treating diabetes in a subject, said method comprising contacting a pancreatic β-cell or a cell which may be induced to become a pancreatic β-cell with a serine or metallo protease inhibitor.

### BRIEF DESCRIPTION OF THE DRAWINGS

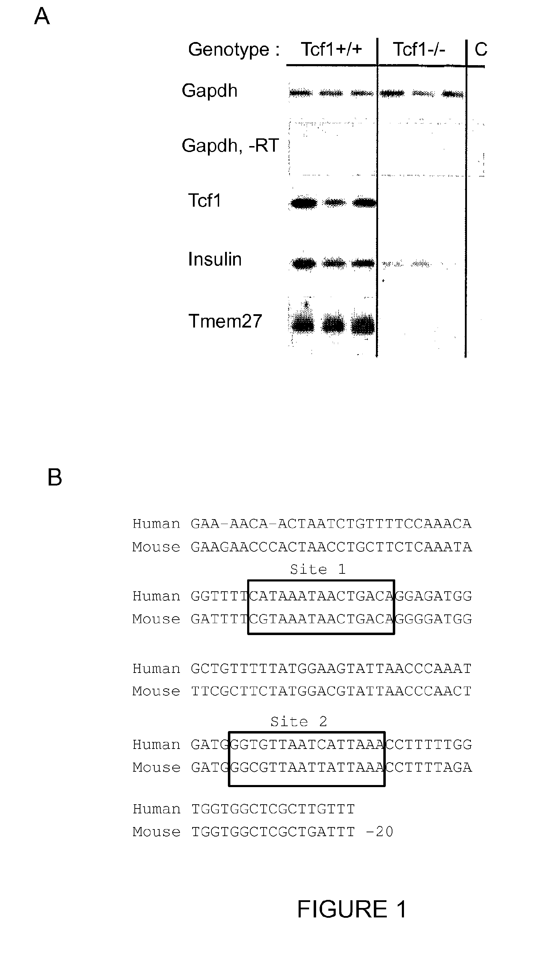

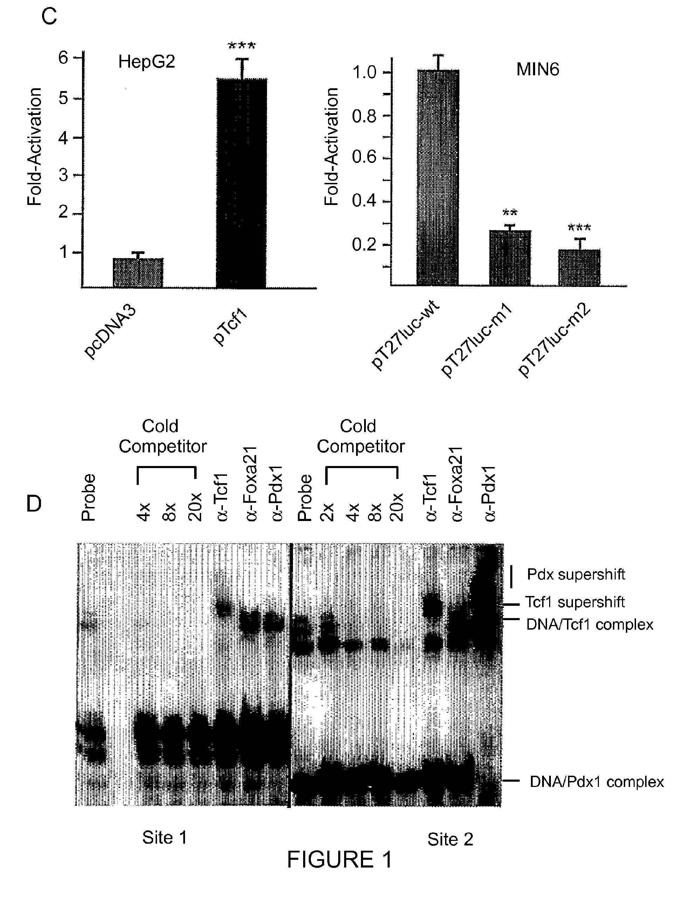

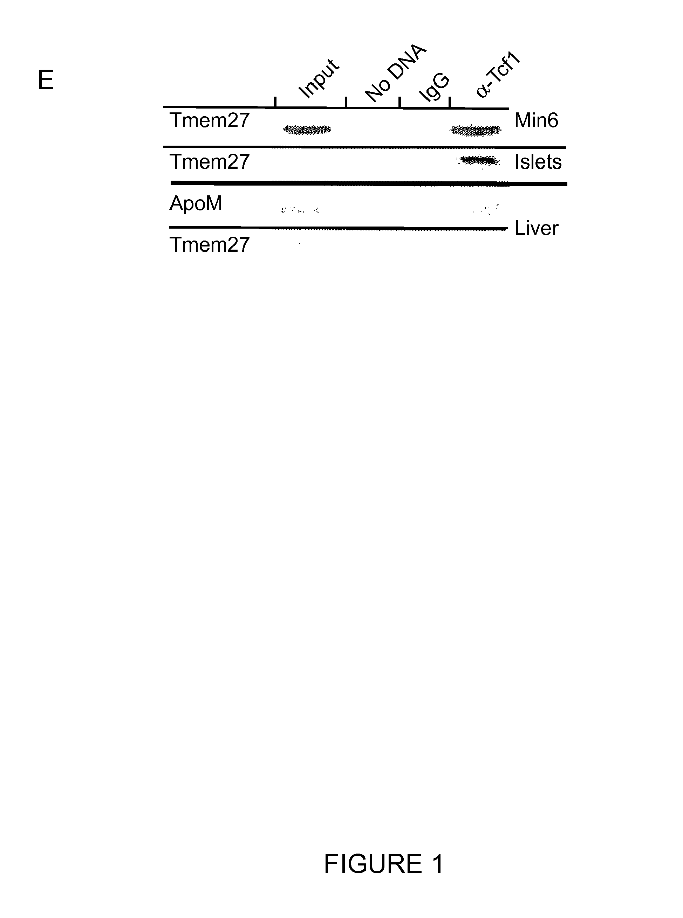

Figure 1 demonstrates TMEM27 expression regulated by Tcf1. **(A)** TMEM27 expression is decreased in the islets of *Tcf1*^{*-*/*-*} mice compared to wildtype littermate controls. Expression levels were measured with RT-PCR, GAPDH was used as a control. **(B)** Promoter analysis predicted two conserved Tcf binding sites in the upstream regulatory regions of human (SEQ ID NO: 10) and mouse (SEQ ID NO: 11) TMEM27 genes, at -60 and -117 base pairs relative to the transcriptional start site. Human and mouse promoter sequences are aligned and Tcf binding sites are boxed in blue. A second site (red box) denotes a putative Pdx-1 binding site. Highlighted sequences (green) indicate positions of the mutated Tcf binding sites. **(C)** Transcriptional activation assay in HepG2 cells that were transfected with pcDNA3 or pTcf1, pCMV-β-glactosidase, and the luciferase reporter gene using a 815 bp TMEM27 mouse promoter. Tcf binding sites were mutated individually in the promoter sequence of the reporter vector and MIN6 cells were transfected with vectors pT27luc-wt, pT27luc-m1 or p27luc-m2. Luciferase activity was normalized to β-galactosidase activity. Each value represents the mean of eight independent experiments. **(D)** EMSA analysis using oligonucleotides for Tcf binding sites 1 and 2 and whole cell extracts of MIN6 cells. Competitor represents unlabeled double-stranded oligonucleotides at a 50-fold excess for binding sites 1 and 2. The dark wedge denotes Tcf1 interaction with the labeled ³²P-labeled probe and the light wedge shows the supershift of this interaction after the addition of an α-Tcf1 antibody. Pdx1 also binds to site 2, as this interaction can be shifted with an anti-Pdx1 antibody.(**E**) ChIP assay in MIN6 cells demonstrates an in vivo interaction of Tcf1 with the promoter of TMEM27. A 150 bp region spanning binding sites 1 and 2 was amplified with PCR. This interaction could not be found in primary hepatocytes which do not express TMEM27. ChIP for Tcf1 was validated in primary hepatocytes by amplification of a region in ApoM promoter that spans the Tcf1 binding site.

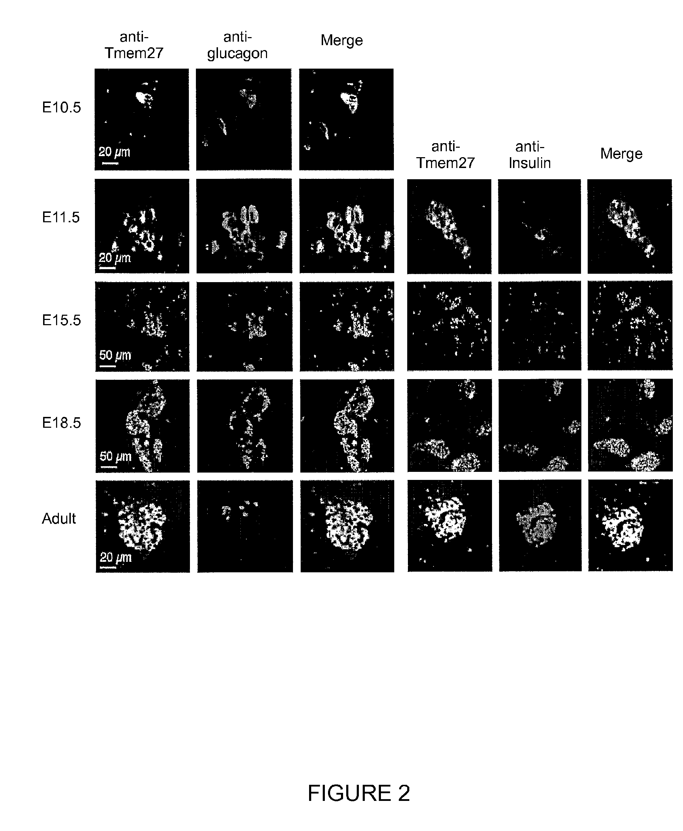

Figure 2 demonstrates TMEM27 expression in the developing mouse pancreas. Immunofluorescence of paraffin embedded pancreatic tissue from denoted time points in the mouse development with anti-Col-4, anti-Insulin and anti-Glucagon antibodies.

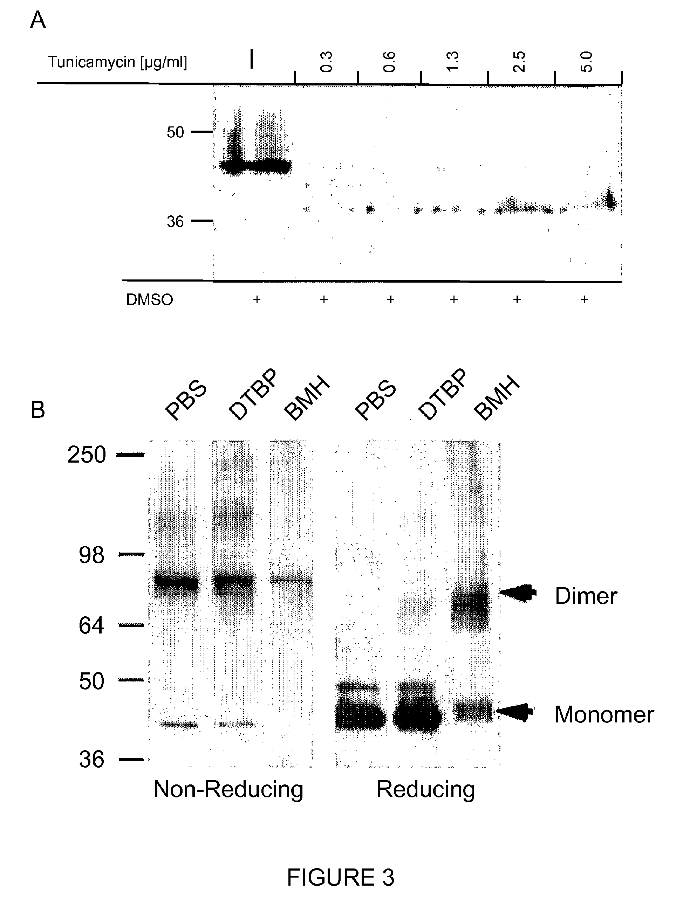

Figure 3 demonstrates that TMEM27 is an N-glycosylated protein and forms dimers. **(A)** MIN6 cells were incubated with increasing amounts of tunicamycin, which inhibits glycosylation at asparagine residues. Addition of DMSO alone to the cells had no effect. **(B)** Supernatant from MIN6 cells was incubated with N-glycanase. The molecular weight shift in the Western blot indicates that the enzyme removed sugar moieties from secreted TMEM27. Secreted protein was detected with anti-Col-3 antibody. **(C)** MIN6 cell lysates were incubated with either PBS or cross-linking reagents DTBP and BMH. SDS-PAGE under non-reducing conditions and immunoblotting using anti-Col-4 antibodies showed a band twice the molecular weight of full-length TMEM27. Under reducing conditions, only the monomer was detected in PBS and DTBP-treated cell lysates. In contrast, the dimer persisted in BMH-treated samples as BMH cross-linking could not be reversed.

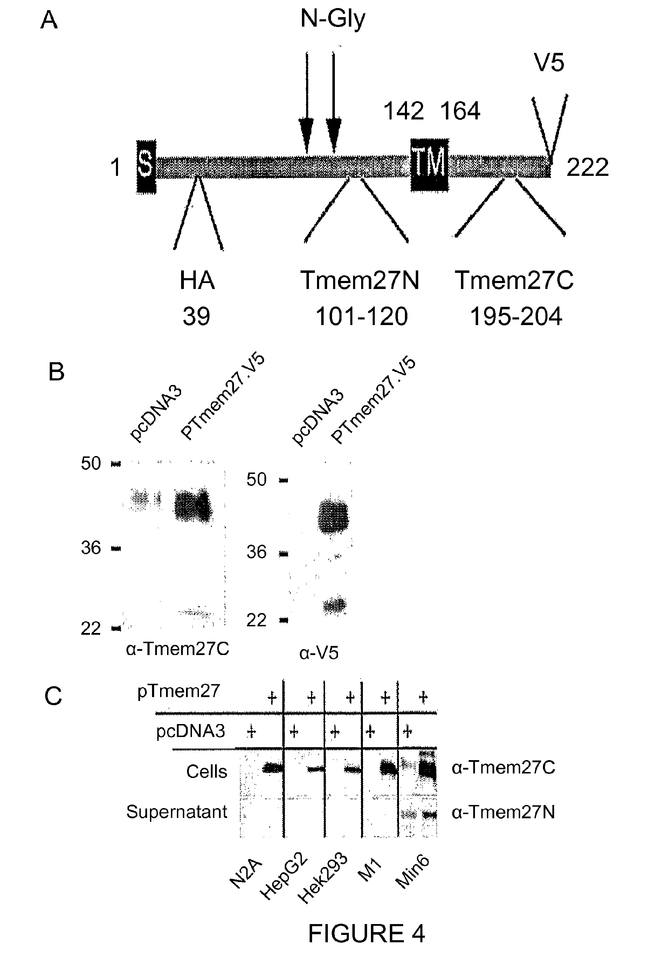

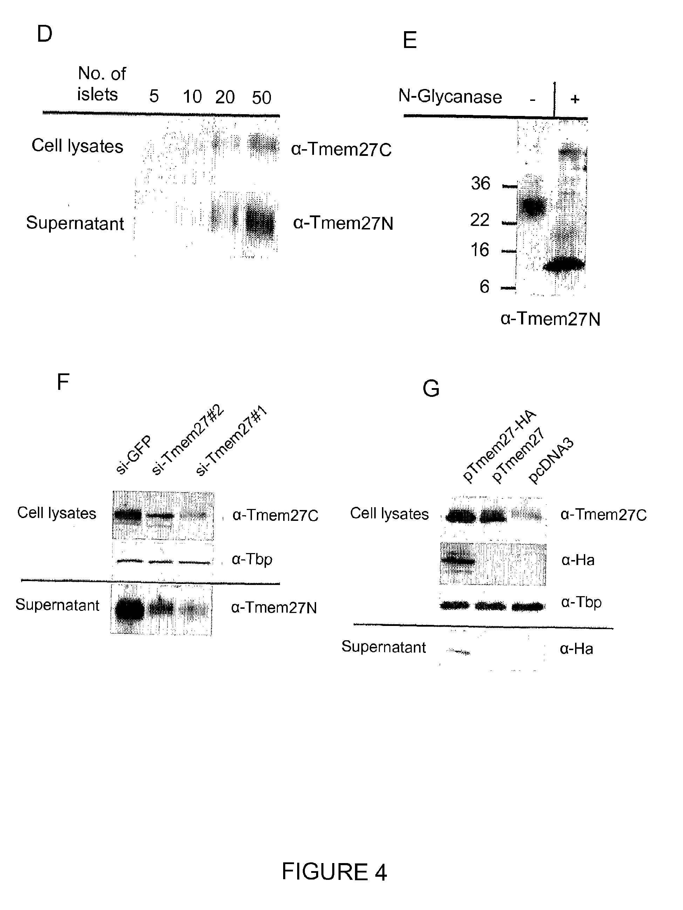

Figure 4 demonstrates that TMEM27 is cleaved and secreted from β-cells of the islet. **(A)** Anti-Col-4 antibody in Western blots of MIN6 cells detected two bands for TMEM27. The 25 kDa band became more abundant in cells that overexpress pTMEM27.V5-His. **(B)** Immunoblots of cell lysates from (A) were probed with α-V5 antibody. The antibody against the V5 epitope also detected both bands in pTMEM27.V5-His transfected cells but not in pcDNA3 transfected cells. **(C)** Denoted cell lines were transfected with pcDNA3 or pTMEM27 and full length protein was detected with anti-Col-4 in Western blots of cell lysates. Secreted protein was detected with anti-Col-3 only in the supernatant of MIN6 cells. Increased expression of full-length protein led to increased amounts of secreted protein solely in the MIN6 cells. **(D)** Mouse pancreatic islets were isolated and incubated for 72 hrs. Supernatants were collected and islets were transferred in lysis buffer. Full-length protein was detected with anti-Col-4 in Western blots of lysates and secreted protein was detected with anti-Col-3 in Western blots loaded with equal volumes of the supernatant. **(E)** MIN6 cells were electroporated with siRNAs targeting GFP or TMEM27. In the cells that were electroporated with si-TMEM27#1 and #2, reduction in the levels of TMEM27 protein was detected with anti-Col-4 48 hrs after electroporation. SDS-PAGE was run with equal volume of supernatants from the same cells and reductions in the levels of secreted proteins were detected with anti-Col-3. **(F)** MIN6 cells were electroporated with pcDNA3, pTMEM27 or pTMEM27-HA. Overexpression of TMEM27 was detected by Western blotting of cell lysates with anti-Col-4 antibodies. Supernatants from the same cells were subjected to Western blotting with anti-HA epitope antibody.

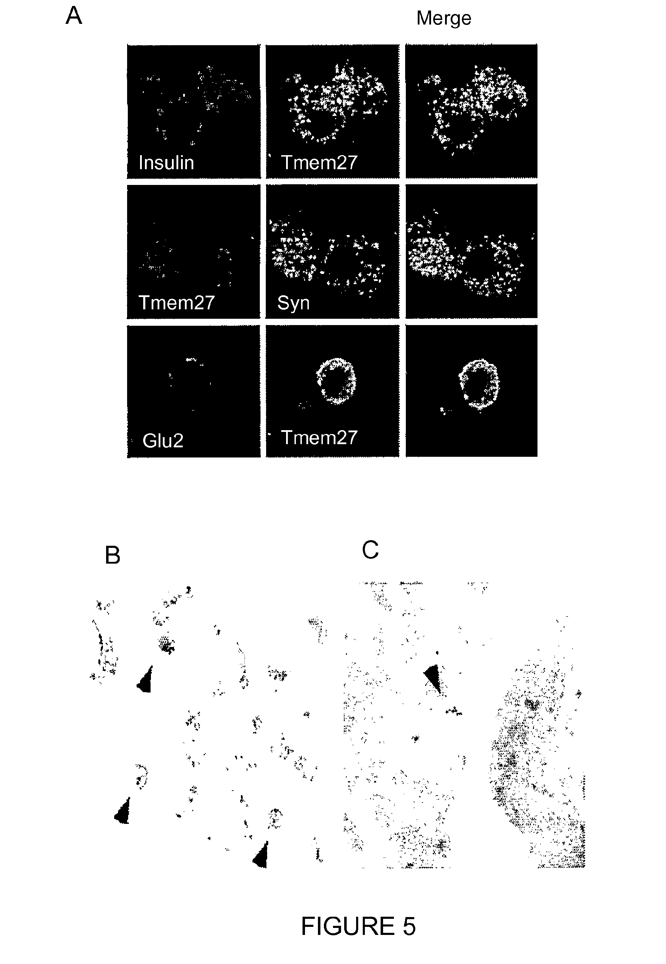

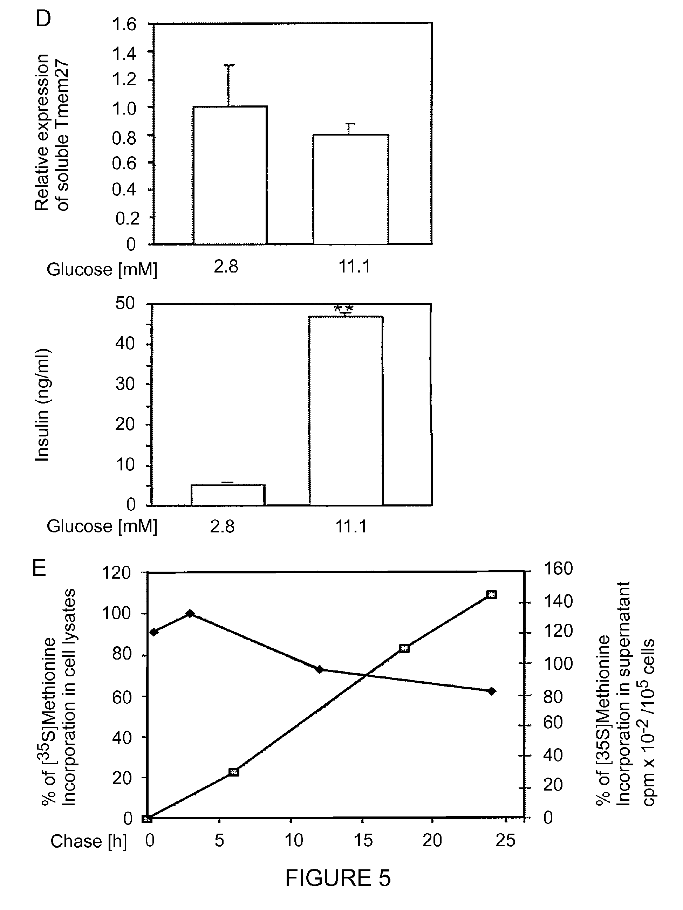

Figure 5 demonstrates that TMEM27 is located in small vesicles and on plasma membranes of pancreatic β-cells. **(A)** MIN6 cells were permeabilized with 0.01% saponin and stained with anti-Col-4 antibodies. Immunofluorescence microscopy with anti-Col-3 antibodies gave similar results. **(B)** Electron microscopy performed on MIN6 cells with anti-Col-4 antibodies localized TMEM27 in small vesicles. IgG conjugated to 10 nm gold particles was used for detection of the antibody. **(C)** Using the same method than in (B), vesicles that contain TMEM27 were detected during fusion events with the plasma membrane.

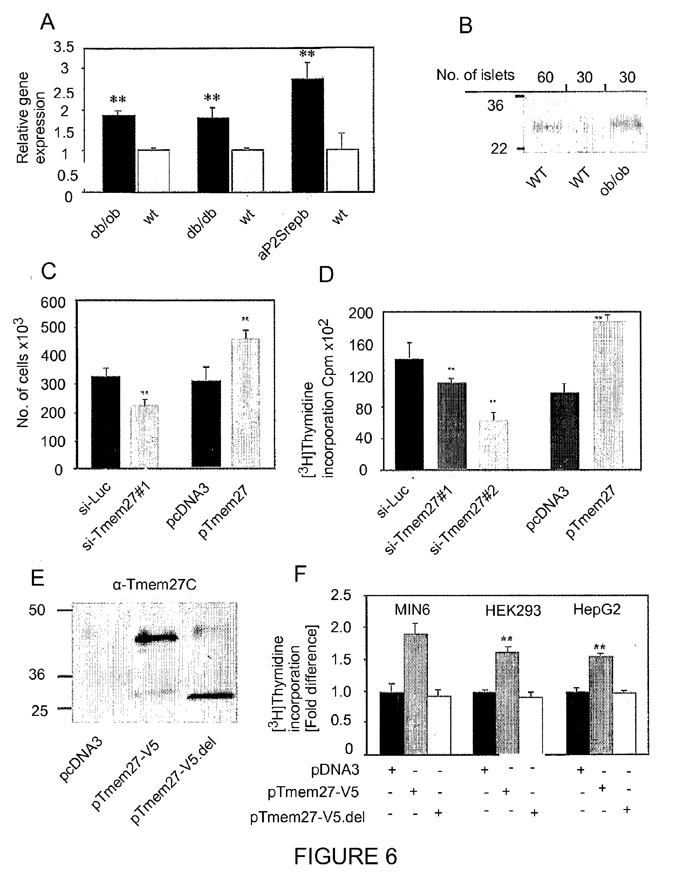

Figure 6 demonstrates TMEM27 increase of β-cell replication *in vitro.* **(A)** TMEM27 expression levels were measured in islets isolated from *ob*/*ob* mice and wildtype littermate controls with semiquantitative RT-PCR. GAPDH was used as a loading control. **(B)** TMEM27 expression levels were measured as above in islets isolated from transgenic *aP-Srebplc* mice and their wildtype littermate controls. **(C)** Isolated islets from wildtype and *ob*/*ob* mice were matched for number and size and incubated in equal volumes of media for 72 hrs. Secreted protein was detected with anti-Col-3. **(D)** MIN6 cells were electroporated with siRNAs targeting luciferase or TMEM27. A representative image of the cells was taken at 20x magnification. Cells were trypsinized 48 hrs after electroporation, stained with trypan blue and counted with a hemocytometer. Each value represents a mean of six independent experiments. **(E)** MIN6 cells were electroporated with pcDNA3 or pTMEM27 and experiments were repeated as above. **(F)** MIN6 cells were electroporated as denoted and cell replication was measured by [³H]thymidine incorporation.

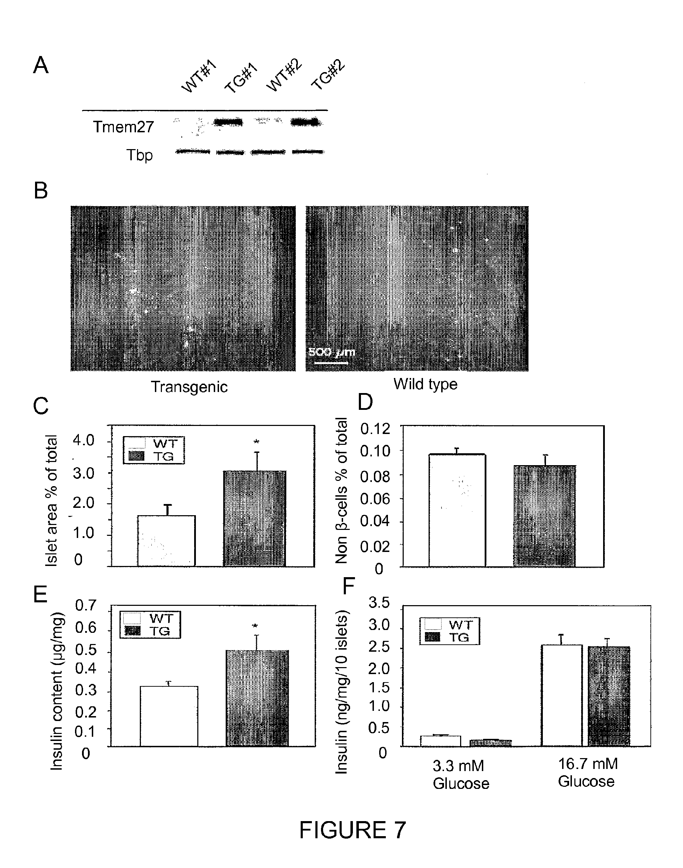

Figure 7 demonstrates increased islet mass in response to increased expression of TMEM27 in pancreatic β-cells. **(A)** Increased expression of TMEM27 was detected with anti-Col-4 in Western blots of islets isolated from RIP-TMEM27 transgenic mice and wildtype littermate controls. **(B)** Whole pancreata from transgenic and control animals (n = 3) were embedded in paraffin and stained for insulin. Images are representative of each genotype at 5x magnification. **(C)** Insulin staining in 7 µm sections was quantified using Metamorph software. At least 50 islets were quantified for each pancreas in sections greater than 200 µm apart. **(D)** Whole pancreata from transgenic and wildtype mice (n = 3) were isolated and homogenized in acid/ethanol. Insulin content was normalized per pancreas weight.

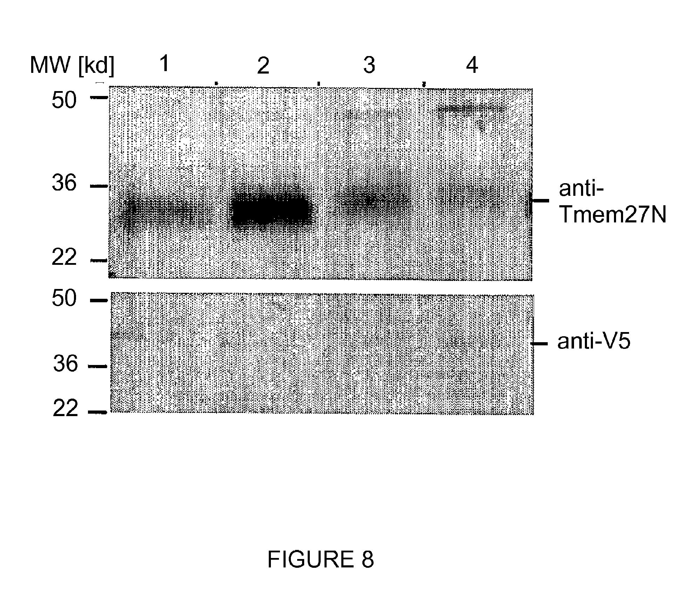

Figure 8 demonstrates that TMEM27 may be a substrate of serine and/or metallo-proteases. MIN6 cells incubated with the serine protease and metalloprotease inhibitor BB94 had lower levels of antibody-recognized cleaved N terminus than MIN6 cells incubated with the phorbol ester phorbol 12-myristate 13-acetate (PMA) or with both BB94+PMA. Cells probed with anti-V5 antibodies, which label the C-terminus of TMEM27, showed comparable bands in all groups.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

This invention provides, in one embodiment, a pancreatic, β-cell specific protein, which participates in β-cell proliferation, and insulin secretion.

As described herein, a frequent form of MODY is caused by mutations in the gene encoding hepatocyte nuclear factor (Tcf1). Mutant mice with loss of Tcf1 function in pancreatic β-cells exhibit progressive hyperglycemia with age due to impaired glucose-stimulated insulin secretion. The mice exhibit a progressive reduction in β-cell number, proliferation rate, and pancreatic insulin content, indicating that Tcf-1 target genes are also required for maintenance of normal β-cell mass. Tcf1 is a direct activator of TMEM27 transcription, as demonstrated herein. TMEM27 is shown herein to be expressed during pancreas development in hormone positive cells and restricted to pancreatic β-cells in the mature pancreas. TMEM27 is shown to be a glycoprotein, specifically cleaved and released by β-cells in the pancreas, and is a stimulator of β-cell replication *in vitro* and *in vivo.*

In one embodiment, this invention provides an isolated nucleic acid comprising a regulatory region of a gene encoding a TMEM27 protein, wherein said regulatory region comprises at least one high-affinity binding site for a Tcf1 protein.

In one embodiment, the term "regulatory region" refers to a promoter, which is a DNA sequence, which, in one embodiment, is upstream of a coding sequence, important for basal and/or regulated transcription of a gene.

In one embodiment, the promoter is an endogenous promoter, comprising at least one high-affinity binding site for a Tcf1 protein. In another embodiment, the promoter is a mutant of the endogenous promoter, which comprises at least one high-affinity binding site for a Tcf1 protein. Mutations in the promoter sequence may enhance Tcf1 binding, or in another embodiment, delay Tcf1 disengagement, or, in another embodiment, via any means, alter Tcf1 binding to the TMEM27 promoter, and thereby influence its transcription. In another embodiment, such mutants will be evaluated for their promoter strength, in terms of the resulting levels of expression of TMEM27.

In one embodiment, the regulatory region has a nucleic acid sequence corresponding to or homologous to SEQ ID NO: 10 or 11.

The nucleic acids used in this invention can be produced by any synthetic or recombinant process such as is well known in the art. Nucleic acids can further be modified to alter biophysical or biological properties by means of techniques known in the art. For example, the nucleic acid can be modified to increase its stability against nucleases (e.g., "end-capping"), or to modify its lipophilicity, solubility, or binding affinity to complementary sequences. These nucleic acids may comprise the vector, the expression cassette, the promoter sequence, the gene of interest, or any combination thereof.

DNA according to the invention can also be chemically synthesized by methods known in the art. For example, the DNA can be synthesized chemically from the four nucleotides in whole or in part by methods known in the art. Such methods include those described in Caruthers (1985). DNA can also be synthesized by preparing overlapping double-stranded oligonucleotides, filling in the gaps, and ligating the ends together (see, generally, Sambrook et al. (1989) and Glover et al. (1995)). DNA expressing functional homologues of the protein can be prepared from wild-type DNA by site-directed mutagenesis (see, for example, Zoller et al. (1982); Zoller (1983); and Zoller (1984); McPherson (1991)). The DNA obtained can be amplified by methods known in the art. One suitable method is the polymerase chain reaction (PCR) method described in Saiki et al. (1988), Mullis et al., U.S. Pat. No.4,683,195, and Sambrook et al. (1989).

In another embodiment, the *Tcf1* binding site comprises a nucleic acid sequence corresponding to or homologous to SEQ ID NO: 12, 13, 14, 15.

In another embodiment, this invention provides a cell, or in another embodiment, a vector comprising a nucleic acid of this invention.

In one embodiment, the term "vector" refers to a nucleic acid construct containing a sequence of interest that has been subcloned within the vector.

To generate the vectors of the present invention, polynucleotides encoding the TMEM27 regulatory region, and in some embodiment, the coding region for TMEM27, and, in some embodiments, other sequences of interest can be ligated into commercially available expression vector systems suitable for transducing/transforming eukaryotic or prokaryotic cells and for directing the expression of recombinant products within the transduced/transformed cells. It will be appreciated that such commercially available vector systems can easily be modified via commonly used recombinant techniques in order to replace, duplicate or mutate existing promoter or enhancer sequences and/or introduce any additional polynucleotide sequences such as for example, sequences encoding additional selection markers or sequences encoding reporter genes.

A vector according to the present invention, may, in another embodiment further include an appropriate selectable marker. The vector may further include an origin of replication, and may be a shuttle vector, which can propagate both in prokaryotic, and in eukaryotic cells, or the vector may be constructed to facilitate its integration within the genome of an organism of choice. The vector, in other embodiments may be, for example, a plasmid, a bacmid, a phagemid, a cosmid, a phage, a virus or an artificial chromosome. In another embodiment, the vector is a viral particle comprising the nucleic acids of the present invention. In another embodiment, this invention provides liposomes comprising the nucleic acids and vectors of this invention. Methods for preparing such liposomes are well known in the art, and may be as described in, for example WO 96/18372; WO 93/24640; Mannino and Gould-Fogerite (1988) BioTechniques 6(7): 682-691; Rose U.S. Pat. No. 5,279,833; WO 91/06309; and Feigner et al. (1987) Proc. Natl. Acad. Sci. USA 84: 7413-7414).

In another embodiment, this invention provides an isolated polypeptide, comprising a secreted form of a TMEM27 protein, wherein said polypeptide is about 25 kDa in size, and comprises an N-terminal fragment of a sequence corresponding to, or homologous to: MLWLLFFLVTAIHAELCQPGAENAFKVRLSIRTALGDKAYAWDTNEEYLFKAMVAFSMRKVPN REATEISHVLLCNVTQRVSFWFVVTDPSKNHTLPAVEVQSAIRMNKNRINNAFFLNDQTLEFLKI PSTLAPPMDPSVPIWIIIFGVIFCIIIVAIALLILSGIWQRRRKNKEPSEVDDAEDKCENMITIENGIPS DPLDMKGGHINDAFMTEDERLTPL (SEQ ID NO: 16). In one embodiment, the secreted form comprises 142 amino acids. In another embodiment, the secreted form comprise the N-terminal 100 amino acids, or in another embodiment, the N-terminal 105 amino acids, or in another embodiment, the N-terminal 110 amino acids, or in another embodiment, the N-terminal 115 amino acids, or in another embodiment, the N-terminal 120 amino acids, or in another embodiment, the N-terminal 125 amino acids, or in another embodiment, the N-terminal 130 amino acids, or in another embodiment, the N-terminal 135 amino acids, or in another embodiment, the N-terminal 140 amino acids, or in another embodiment, the N-terminal 122 amino acids, or in another embodiment, the N-terminal 127 amino acids, or in another embodiment, the N-terminal 117 amino acids, or in another embodiment, the N-terminal 108 amino acids.

In one embodiment, the TMEM27 protein has a sequence corresponding to, or homologous to one disclosed in NCBI's Entrez protein database, having the Accession numbers: NP_065651, AAH50606, AAH15099, XP_416821, AAH49912, or AAH14317. In one embodiment, the polypeptides of this invention will comprise amino acids corresponding to those at positions 1-142 of a TMEM27 protein, or, in another embodiment, an N-terminal fragment thereof, or in another embodiment, a homologue thereof.

In one embodiment, the terms "homology", "homologue" or "homologous", in any instance, indicate that the sequence referred to, whether an amino acid sequence, or a nucleic acid sequence, exhibits at least 70 % correspondence with the indicated sequence. In another embodiment, the amino acid sequence or nucleic acid sequence exhibits at least 72 % correspondence with the indicated sequence. In another embodiment, the amino acid sequence or nucleic acid sequence exhibits at least 75 % correspondence with the indicated sequence. In another embodiment, the amino acid sequence or nucleic acid sequence exhibits at least 77 % correspondence with the indicated sequence. In another embodiment, the amino acid sequence or nucleic acid sequence exhibits at least 80 % correspondence with the indicated sequence. In another embodiment, the amino acid sequence or nucleic acid sequence exhibits at least 82 % correspondence with the indicated sequence. In another embodiment, the amino acid sequence or nucleic acid sequence exhibits at least 85 % correspondence with the indicated sequence. In another embodiment, the amino acid sequence or nucleic acid sequence exhibits at least 87 % correspondence with the indicated sequence. In another embodiment, the amino acid sequence or nucleic acid sequence exhibits at least 90 % correspondence with the indicated sequence. In another embodiment, the amino acid sequence or nucleic acid sequence exhibits at least 92 % correspondence with the indicated sequence. In another embodiment, the amino acid sequence or nucleic acid sequence exhibits at least 95 % or more correspondence with the indicated sequence. In another embodiment, the amino acid sequence or nucleic acid sequence exhibits 95 % - 100 % correspondence to the indicated sequence. Similarly, as used herein, the reference to a correspondence to a particular sequence includes both direct correspondence, as well as homology to that sequence as herein defined.

The term "polypeptide" refers, in one embodiment, to a protein or, in another embodiment, protein fragment, or, in another embodiment, a string of amino acids. In one embodiment, reference to "peptide" or "polypeptide" when in reference to any polypeptide of this invention, is meant to include native peptides (either degradation products, synthetically synthesized peptides or recombinant peptides) and peptidomimetics (typically, synthetically synthesized peptides), such as peptoids and semipeptoids which are peptide analogs, which may have, for example, modifications rendering the peptides more stable while in a body or more capable of penetrating into cells. Such modifications include, but are not limited to N terminal, C terminal or peptide bond modification, including, but not limited to, backbone modifications, and residue modification, each of which represents an additional embodiment of the invention. Methods for preparing peptidomimetic compounds are well known in the art and are specified, for example, in Quantitative Drug Design, C.A. Ramsden Gd., Chapter 17.2, F. Choplin Pergamon Press (1992).

It is to be understood that any amino acid sequence whether obtained naturally or synthetically, by any means, exhibiting sequence, structural, or functional homology to the polypeptides described herein are considered as part of this invention.

In one embodiment, the term "homology", when in reference to any nucleic acid sequence indicates a percentage of nucleotides in a candidate sequence that are identical with the nucleotides of a corresponding native nucleic acid sequence.

Homology may be determined by computer algorithm for sequence alignment, by methods well described in the art. For example, computer algorithm analysis of nucleic acid or amino acid sequence homology may include the utilization of any number of software packages available, such as, for example, the BLAST, DOMAIN, BEAUTY (BLAST Enhanced Alignment Utility), GENPEPT and TREMBL packages.

An additional means of determining homology for nucleic acids is via determination of candidate sequence hybridization, methods of which are well described in the art (See, for example, "Nucleic Acid Hybridization" Hames, B. D., and Higgins S. J., Eds. (1985); Sambrook et al., 1989, Molecular Cloning, A Laboratory Manual, (Volumes 1-3) Cold Spring Harbor Press, N.Y.; and Ausubel et al., 1989, Current Protocols in Molecular Biology, Green Publishing Associates and Wiley Interscience, N.Y). For example methods of hybridization may be carried out under moderate to stringent conditions, to the complement of a DNA encoding a native caspase peptide. Hybridization conditions being, for example, overnight incubation at 42 °C in a solution comprising: 10-20 % formamide, 5 X SSC (150 mM NaCl, 15 mM trisodium citrate), 50 mM sodium phosphate (pH 7. 6), 5 X Denhardt's solution, 10 % dextran sulfate, and 20 µg/ml denatured, sheared salmon sperm DNA.

In another embodiment, this invention provides an antibody specifically recognizing a polypeptide of this invention. Production of such an antibody is well known in the art, and may comprise methods as described in, for example, Harlow and Lane, Antibodies: A Laboratory Manual, Cold Spring Harbor Laboratory, New York, 1988. In one embodiment, such antibodies may comprise functional fragments, which are well known to those skilled in the art, and may be prepared for example, by proteolytic hydrolysis of the antibody or by expression in E. coli or mammalian cells (e.g. Chinese hamster ovary cell culture or other protein expression systems) of DNA encoding the fragment.

In another embodiment, this invention provides compositions comprising the cells, polypeptides, antibodies, nucleic acids, and vectors of this invention. The effective dose and method of administration of a particular composition formulation can vary based on the particular application. Dosage may vary as a function of the type of vector, for example, employed and the route of administration.

In another embodiment, this invention provides cells comprising the nucleic acids, vectors, or polypeptides of this invention. In one embodiment, the cells are prokaryotic, or in another embodiment, the cells are eukaryotic. Cells may be any cells capable of expressing the isolated nucleic acids and/or vectors of this invention, as will be known to one skilled in the art. In one embodiment, cells may overexpress the polypeptides of this invention.

In another embodiment, cells are responsive to the polypeptides of this invention. In one embodiment, the cells are pancreatic β cells, or a pancreatic β cell, or a cell that may differentiate into a pancreatic β cell. In one embodiment, the cells, upon exposure to TMEM27, which, in one embodiment, is the full-length protein, or in another embodiment, is the cleaved secreted form, proliferate, or, in another embodiment, secrete insulin, or in another embodiment, do both. In one embodiment, the cells are terminally differentiated, or, in another embodiment, non-dividing. In another embodiment, the cells are dividing.

In one embodiment, this invention provides nucleic acids, vectors, polypeptides, cells, and compositions comprising the same, which may, in another embodiment, be formulated for oral or, in another embodiment, local administration, such as by aerosol, intramuscularly or transdermally, or via parenteral application. Compositions can be administered in a variety of unit dosage forms depending upon the method of administration. Suitable unit dosage forms, include, but are not limited to powders, tablets, pills, capsules, lozenges, suppositories, etc. Transdermal administration may be accomplished by application of a cream, rinse, gel, etc. capable of allowing the active compounds to penetrate the skin. Parenteral routes of administration may include, but are not limited to, electrical or direct injection such as direct injection into a central venous line, intravenous, intramuscular, intraperitoneal, intradermal, or subcutaneous injection.

TMEM27 expression was demonstrated herein in β-cells of the islet in newborn and adult pancreas (Figure 2), and an N-terminal cleavage product of the TMEM27 protein was found to be secreted (Figure 4). β-cell lines proliferated in response to TMEM27 (Figure 6), and transgenic mice overexpressing the protein showed increased β-cell mass as a result of expression of the transgene (Figure 7).

In one embodiment, this invention provides a method of increasing pancreatic β-cell mass, where the method comprises contacting a cell with a TMEM27 polypeptide. According to this aspect of the invention, and in one embodiment, the cell is a pancreatic β-cell or said cell may be induced to become a pancreatic β-cell and providing conditions favorable for β-cell proliferation.

In one embodiment, the term "contacting a cell" refers to any exposure of a cell to a peptide, nucleic acid, or composition of this invention. Cells may be in direct contact with compounds and compositions of the invention, or exposed indirectly, through methods well described in the art. For example, cells grown in media in vitro, wherein the media is supplemented with polypeptides, nucleic acids, vectors or compositions of this invention would be an example of a method of contacting a cell, and considered a part of this invention. In another embodiment, contacting a cell may include any route of administration to a subject, for example, oral or parenteral administration of a peptide, nucleic acid, vector or composition of this invention to a subject, wherein administration results in *in vivo* cellular exposure to these materials, within specific sites within a body.

In one embodiment, the cells being contacted are primary cultures. In one embodiment, "primary culture" denotes a mixed cell population that permits interaction of many different cell types isolated from a tissue. For example, a primary culture of pancreatic duct cells may allow the interaction between mesenchymal and epithelial cells.

In another embodiment, a primary culture may be a purified cell population isolated from a tissue. In one embodiment, the primary culture may be enriched for a particular population. In one embodiment, enrichment may comprise cell sorting via means well known in the art, such as, for example fluorescent activated cell sorting (FACS), for cell populations, for example, expressing a particular cell surface marker, or in another embodiment, lacking cell surface expression of a particular marker. In another embodiment, manetoseparation methods may be used, or in another embodiment, density centrifugation methods, such as, for example, ficoll-hypaque or sucrose gradient separation.

In one embodiment, the cells contacted according to the methods of this invention are stem or progenitor cells. The term "progenitor cell" is used synonymously with "stem cell", and refers, in some embodiments, to an undifferentiated cell which is capable of proliferation and giving rise to more progenitor cells having the ability to generate a large number of mother cells that can, in turn, give rise to differentiated, or differentiable daughter cells. In one embodiment, the term progenitor or stem cell refers to a generalized mother cell whose descendants (progeny) specialize, often in different directions, by differentiation, e.g., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. It is to be understood that any such cells may be used for the methods of this invention, or comprise the cells of this invention. Such capacity may be natural or may be induced artificially upon treatment with various factors, either scenario is to be considered as an embodiment of the cells which may be used according to the methods of this invention, or comprising a cell of this invention.

In one embodiment, cells of this invention and in use for the methods of this invention derive from the pancreas, or differentiate to a pancreatic cell. In one embodiment, the term "pancreas" refers generally to a large, elongated, racemose gland situated transversely behind the stomach, between the spleen and duodenum. In one embodiment, the cells of this invention may comprise tissue slices, or whole tissue, which comprise the polypeptides, nucleic acids, vectors or compositions, as described herein. In another embodiment, the methods of this invention include organ cultures, or, in another embodiment, tissue slices, or tissue fragments, comprising cell populations of interest. In one embodiment, islets of Langerhans are used as the cells and for the methods of this invention. In one embodiment, the cell is a β cell, and may constitute 60-80% of the islet cells, and/or may be used in the methods of this invention.

In one embodiment, the cell is a pancreatic progenitor cell. In one embodiment, the phrase "pancreatic progenitor cell" refers to a cell which can differentiate into a cell of pancreatic lineage, e.g. a cell which can produce a hormone or enzyme normally produced by a pancreatic cell. For instance, a pancreatic progenitor cell may be caused to differentiate, at least partially, into α, β, γ, or δ islet cell, or a cell of exocrine fate. The pancreatic of progenitor cells of the invention can also be cultured prior to administration to a subject under conditions which promote cell proliferation and differentiation. These conditions include culturing the cells to allow proliferation and confluence in vitro at which time the cells can be made to form pseudo islet-like aggregates or clusters and secrete insulin, glucagon, and/or somatostatin.

In one embodiment, the cells of this invention and for use in the methods of this invention, may be a substantially pure population. In one embodiment, the term "substantially pure", refers to a population of cells that is at least about 65%, or, in another embodiment, at least about 70%, or, in another embodiment, at least about 75%, or, in another embodiment, at least about 85%, or, in another embodiment, at least about 90%, or, in another embodiment, at least about 95% pure, with respect to the total cell population.

Various techniques may be employed to obtain suspensions of cells (both differentiated and undifferentiated) from tissues, to comprise the cells of this invention and/or for use in the methods of this invention. In some embodiments, isolation procedures are ones that result in as little cell death as possible. For example, the cells can be removed from an explant sample by mechanical means, e.g., mechanically sheared off with a pipette. In other instances, it will be possible to dissociate the progenitor cells from the entire explant, or sub-portion thereof, e.g., by enzymatic digestion of the explant, followed by isolation of the activated progenitor cell population based on specific cellular markers, e.g., using affinity separation techniques or fluorescence activated cell sorting (FACS). Cells may be obtained from liquid samples, such as blood, by centrifugation.

In general, the tissue is prepared using any suitable method, such as by gently teasing apart the excised tissue or by digestion of excised tissue with collagenase (for example, collagenase A), via, to illustrate, perfusion through a duct or simple incubation of, for example, teased tissue in a collagenase-containing buffer of suitable pH and tonic strength. The prepared tissue may then, optionally, be concentrated using suitable methods and materials, such as centrifugation through Ficol gradients for concentration (and partial purification). The concentrated tissue then is resuspended into any suitable vessel, such as tissue culture glassware or plasticware. In certain embodiments, the sample pancreatic tissue is allowed to form a confluent monolayer culture, from which NACs are formed. In other preferred embodiments, the cell suspension is placed in a non-adherent culture container and spheres of progenitor cells are formed.

In one embodiment, tissue from which the cells are derived may be adult or fetal tissue or tissue from any developmental stage. Moreover, the method can be practiced with relatively small amounts of starting material. Accordingly, small samples of tissue from a donor can be obtained without sacrificing or seriously injuring the donor. The progenitor cells of the present invention can be amplified, and subsequently isolated from a tissue sample.

In certain embodiments, the culture may be contacted with a growth factor or a composition comprising a growth factor, e.g., a mitogenic growth factor, e.g., the growth factor is selected from a group consisting of IGF-I, IGF-II, LIF, TGFα, TGFβ, bFGF, aFGF, HGF or hedgehog. In other embodiments, the growth factor is a member of the TGFβ superfamily. In some embodiments, the methods of this invention comprise administering a composition to the subject, as described, wherein the composition may comprise a cell, polypeptide, nucleic acid and/or vector of this invention, further comprising any additive as herein described, including, for example, a diabetes treatment, a growth factor, a cAMP elevating agent, etc.

In some embodiments, the cells, or cells in culture are contacted with, or composition comprises, a cAMP elevating agents, such as 8-(4-chlorophenylthio)-adenosine-3':5'-cyclic-monophosphate (CPT-cAMP) (see, for example, Koike Prog. Neuro-Psychopharmacol. and Biol. Psychiat. 16 95-106 (1992)), CPT-cAMP, forskolin, Na-Butyrate, isobutyl methylxanthine (IBMX) and cholera toxin (see Martin et al. J. Neurobiol. 23 1205-1220 (1992)) and 8-bromo-cAMP, dibutyryl-cAMP and dioctanoyl-cAMP (e.g., see Rydel et al. PNAS 85:1257 (1988)).

In some embodiments, the cells, or cells in culture are contacted with, or composition comprises, a steroid or corticosteroid such as, for example, hydrocortisone, deoxyhydrocortisone, fludrocortisone, prednisolone, methylprednisolone, prednisone, triamcinolone, dexamethasone, betamethasone and paramethasone. See, generally, The Merck Manual of Diagnosis and Therapy, 15th Ed., pp. 1239-1267 and 2497-2506, Berkow et al., eds., Rahway N.J., 1987).

There are a large number of tissue culture media that exist for culturing tissue from animals, including humans. In some embodiments, culture medium may be a simple medium, such as Dulbecco's Minimal Essential Media (DMEM). In another embodiment, cells, tissues, etc., are cultured in Isocove's modified MEM cell culture medium with 5% FBS. In another embodiment, cells, tissue, etc., are maintained in the absence of sera for extended periods of time. In some embodiments of the invention, the growth factors or other mitogenic agents are not included in the primary media for maintenance of the cultures in vitro, but are used subsequently to cause proliferation of distinct populations of cells, such as, for example, progenitor cells.

In another embodiment, stem cells are enriched because of their ability to grow in the absence of adherence, either direct or indirect, to a culture surface. In some embodiments, stem cells are free floating in suspension and are not in fixed or semi-fixed contact with a surface of the culture vessel or with other cells or materials that are in contact.

In one embodiment, the cells of this invention are for use in, and methods of this invention provide a source of implantable cells, either in the form of the progenitor cell population of the differentiated progeny thereof, or in another embodiment, the subject cells can be used to produce cultures of pancreatic cells for production and purification of secreted factors, such as the secreted form, for example, of TMEM27. In another embodiment, cultured cells can be provided as a source of insulin.

In another embodiment, this invention provides a method of increasing pancreatic β-cell mass, the method comprising contacting a cell with a nucleic acid encoding a TMEM27 polypeptide, wherein said cell is a pancreatic β-cell or said cell may be induced to become a pancreatic β-cell and providing conditions favorable for expression of said nucleic acid.

In one embodiment, increased β-cell mass occurs via increased proliferation of β-cells. In one embodiment, increased β-cell mass results from enhanced differentiation of precursor cells to a β-cell lineage. In one embodiment, increased β-cell mass refers to diminished cell turnover or death. In another embodiment, increased β-cell mass refers to any combination of the aforementioned embodiments.

Cell proliferation may be determined via any number of methods well known in the art, and as exemplified herein, for example via measuring uptake of a labeled substrate, such as tritiated thymidine, as will be known to one skilled in the art.

In another embodiment, this invention provides a method of altering metabolism in a subject, the method comprising contacting a cell with a TMEM27 polypeptide or a nucleic acid encoding said TMEM27 polypeptide, wherein said cell is a pancreatic β-cell or said cell may be induced to become a pancreatic β-cell.

In one embodiment, altering metabolism refers to increasing metabolism, while in another embodiment, it referes to decreasing metabolism. In one embodiment, glucose metabolism is altered. In one embodiment, the cell is contacted *in vitro* or *ex vivo.* In another embodiment, the cell is contacted *in vivo.* In another embodiment, the cell is a stem or progenitor cell. In another embodiment, the cell is isolated from a subject suffering from or predisposed to diabetes.

In one embodiment, the method comprises the step of administering the contacted cell to the subject, such as, for example, ex-vivo cellular therapy. In one embodiment, cells administered to a subject will be autologous or, in another embodiment, allogeneic with respect to the subject.

In one embodiment, the cells are contacted with a TMEM27 polypeptide which comprises a full-length protein, or a fragment thereof. In one embodiment, the fragment comprises a secreted form of a TMEM27 protein. In another embodiment, the secreted form is about 25 kDa in size, and comprises amino acids 1-142 of the TMEM27 protein, or a fragment thereof, or a sequence homologous thereto.

In one embodiment, the cells may also be contacted with a protease inhibitor. In one embodiment, a protease inhibitor is a molecule which represses, prevents, diminishes, delays, or in any way alters protease activity or expression or both.

In one embodiment, the protease inhibitor is a serine protease inhibitor (serpin), a metallo protease inhibitor, a cysteine protease inhibitor, a thio protease inhibitor, a trypsin inhibitor, a threonine protease inhibitor, an aspartic protease inhibitor, or a combination thereof.

In one embodiment, the protease inhibitor is (HONH-COCH2CH2CO-FA-NH2), (OA-Hy; cis-9-Octadecenoyl-N-hydroxylamide; Oleoyl-N-hydrocylamide), {N-[[(4,5-Dihydro-5-thioxo-1,3,4-thiadiazol-2-yl)amino]carbonyl]-L-phenylalanine Methyl Ester}, {a-[[[4,5-Dihydro-5-thioxo-1,3,4-thiadiazol-2-yl)amino]carbonyl]amino]- ((2-pyridyl)piperazinyl)-(S)- benzenepropanamide}, {(2R)-2-[(4-Biphenylylsulfonyl)amino]-3-phenylpropionic Acid}, {(2R)-[(4-Biphenylylsulfonyl)amino]-N-hydroxy-3-phenylpropionamide}, H-Cys1-Thr-Thr-His-Trp-Gly-Phe-Thr-Leu-Cys10-OH, (SB-3CT), (Ac-RCGVPD-NH2; Stromelysin-1 Inhibitor), [N-Isobutyl-N-(4-methoxyphenylsulfonyl)-glycylhydroxamic Acid; NNGH] {N-[[4,5-Dihydro-5-thioxo-1,3,4-thiadiazol-2-yl)amino]carbonyl]-L-phenylalanine}, {a-[[[4,5-Dihydro-5-thioxo-1,3,4-thiadiazol-2-yl)amino]carbonyl]amino]-N-(cyclohexylmethyl)-(S)-benzenepropanamide}, 4-Dibenzofuran-2¢-yl-4-hydroximino-butyric Acid), [4-(4¢-Biphenyl)-4-hydroxyimino-butyric-Acid], {(3R)-(+)-[2-(4-Methoxybenzenesulfonyl)-1,2,3,4-tetrahydroisoquinoline-3-hydroxamate]}, {(3S)-(-)-[2-(4-Methoxybenzenesulfonyl)-1,2,3,4-tetrahydroisoquinoline-3-hydroxamate]}, [N-Hydroxy-1-(4-methoxyphenyl)sulfonyl-4-(4-biphenylcarbonyl)piperazine-2-carboxamide], [N-Hydroxy-1-(4-methoxyphenyl)sulfonyl-4-benzyloxycarbonylpiperazine-2-carboxamide], (1,10-Phenanthroline Monohydrate), Marimastat, Prinomastat, Tanomastat, Neovastat, Zoledronic acid, or a combination thereof.

In one embodiment, the inhibitor is Alpha 1-antitrypsin, Alpha 1-antichymotrypsin, Alpha 2-antiplasmin (which in one embodiment, is an inhibitor of fibrinolysis), Antithrombin (which in one embodiment, is an inhibitor of coagulation, specifically factor X, factor IX and thrombin), Complement 1-inhibitor, Neuroserpin (which in one embodiment, is mutated in some familial forms of dementia), Plasminogen activator inhibitor-1 and 2 (which in one embodiment, is an inhibitor of fibrinolysis), Protein Z-related protease inhibitor (ZPI, which in one embodiment, inactivates factor Xa and factor XIa), or a combination thereof.

In another embodiment, the protease inhibitor is Leupeptin, 4-(2-aminoethyl)benzenesulfonyl fluoride hydrochloride (AEBSF), Aprotinin, Chymostatin, Antithrombin III, 3,4-Dichloroisocoumarin, L-1-chloro-3-[4-tosyl-amido]-7-amino-2-heptanone-HCl (TLCK), TPCK, diisopropyl phosphorofluoridate (DIFP), Antipain, 4-amidinophenylmethanesulfonyl-fluoride-HCl (APMSF), phenylmethanesulfonyl fluoride (PMSF), 3,4-dichloroisocoumarin (DCI), α-toluenesulfonyl fluoride, BB94, α2-Macroglobulin, or a combination thereof.

In one embodiment, the protease inhibitor is EDTA, vanadium, molybdate salts, 1,10-Phenanthroline, Phosphoramidon, amastatin, Bestatin, actinonin, diprotin, BB94, α2-Macroglobulin, or a combination thereof.

In one embodiment, the protease inhibitor is N-Ethylmaleimide, Leupeptin, L-transepoxy-succinyl-leucyl-amido-(4-guanidino)-butane (E-64), Chymostatin, Antipain, α2-Macroglobulin, PMSF, PEFABLOC, or a combination thereof.

In one embodiment, the protease inhibitor is Pepstatin A, α2-Macroglobulin, or a combination thereof. In another embodiment, the protease inhibitor is BB94 or batimastat, which in one embodiment is a serine or metallo protease inhibitor. In one embodiment, the protease inhibitor is reversible, while in another embodiment, the protease inhibitor is irreversible.

In another embodiment, the cells may also be contacted with an inhibitor of PKC, which in one embodiment is Safingol (L-threo-dihydrosphingosine), Ro-1, Ro32-0432 (Bisindolylmaleimide tertiary amine), UCN-01 (7-OH-staurosporine), Flavopiridol (L86-8275), Bryostatin 1 (Macrocyclic lactone), Bisindolylmaleimide I, InSolution™ Bisindolylmaleimide I, Bisindolylmaleimide I, Hydrochloride, Bisindolylmaleimide II, Bisindolylmaleimide III, Hydrochloride, Bisindolylmaleimide Inhibitor Set, Bisindolylmaleimide IV, Bisindolylmaleimide V, Calphostin C, *Cladosporium cladosporioides,* Cardiotoxin, *Naja nigricollis,* Chelerythrine Chloride, Dequalinium Chloride, Ellagic Acid, Dihydrate, G6976, InSolution™ G6976, G6983, G7874, Hydrochloride, H-7, Dihydrochloride, Iso-H-7, Dihydrochloride, HBDDE, Hispidin, Hypericin, K-252a, *Nocardiopsis* sp., InSolution™ K-252a, *Nocardiopsis* sp., K-252b, *Nocardiopsis* sp., K-252c, Melittin, NGIC-I, Phloretin, Piceatannol, PKC_{bII}/EGFR Inhibitor, Staurosporine, N-Benzoyl PKC_{b} Inhibitor, Polymyxin B Sulfate, Protein Kinase C Inhibitor 20-28, Cell-Permeable, Myristoylated, Protein Kinase C Inhibitor Peptide 19-31, Protein Kinase C Inhibitor Peptide 19-36, Protein Kinase C Inhibitor Set, Protein Kinase C Inhibitor, EGF-R Fragment 651-658, Myristoylated, Protein Kinase Cₑ Translocation Inhibitor Peptide, Protein Kinase Cₑ Translocation Inhibitor Peptide, Negative Control, Protein Kinase C_{z} Pseudosubstrate Inhibitor, Protein Kinase C_{z} Pseudosubstrate Inhibitor, Myristoylated, Protein Kinase Cₕ Pseudosubstrate Inhibitor, Myristoylated, Protein Kinase Cq Pseudosubstrate Inhibitor, Protein Kinase C_{q} Pseudosubstrate Inhibitor, Myristoylated, Pseudohypericin, Ro 31-7549, Immobilized, Ro-31-7549, Ro-31-8220, InSolution™ Ro-31-8220, Ro-31-8425, Ro-32-0432, Rottlerin, Safingol, Sangivamycin, Scytonemin, *Lyngbya* sp., Serine/Threonine Kinase Inhibitor Set, D-*erythro*-Sphingosine, Dihydro-, D-*erythro*-Sphingosine, Free Base, Bovine Brain, D-*erythro*-Sphingosine, Free Base, High Purity, D-*erythro*-Sphingosine, N,N-Dimethyl-, Staurosporine, *Streptomyces* sp., Tamoxifen Citrate, Tamoxifen, 4-Hydroxy-, (Z)-, TER14687, Vitamin E Succinate, or antisense nucleotides capable of inhibiting the expression of the protein kinase C.

In one embodiment, the protease comprise trypsin, chymotrypsin and elastase. In one embodiment, cysteine proteases comprise papain, calpain and lysosomal cathepsins. In one embodiment, aspartic proteases include pepsin and rennin. In one embodiment, Metallo-proteases include thermolysin and carboxypeptidase A.

In one embodiment, the compositions and/or methods of this invention may comprise use of any combination of inhibitors as herein described.

In another embodiment, the cells may also be contacted with a Tcf1 protein or a nucleic acid encoding said Tcf1 protein.

In one embodiment, the Tcf1 protein may have an amino acid sequence, which is homologous to, or as set forth in NCBI's Genbank, Accession Number: CAI59557, P15257, P22361, P20823, NP_033353, NP_036801, NP_739570 or NP_000536.

In another embodiment, this invention provides a method of increasing pancreatic β-cell mass, the method comprising contacting a pancreatic β-cell or a cell which may be induced to become a pancreatic β-cell with a serine or metallo protease inhibitor, wherein said cell expresses or may be induced to express TMEM27 and providing conditions favorable for β-cell proliferation.

In another embodiment, this invention provides a method of altering metabolism in a subject, the method comprising contacting a pancreatic β-cell or a cell which may be induced to become a pancreatic β-cell with a serine or metallo protease inhibitor, wherein said cell expresses or may be induced to express TMEM27.

In another embodiment, this invention provides a method of inhibiting, suppressing or treating diabetes in a subject, the method comprising contacting a cell with a TMEM27 polypeptide or a nucleic acid encoding said TMEM27 polypeptide, wherein said cell is a pancreatic β-cell or said cell may be induced to become a pancreatic β-cell.

In another embodiment, this invention provides a method of inhibiting, suppressing or treating diabetes in a subject, the method comprising contacting a pancreatic β-cell or a cell which may be induced to become a pancreatic β-cell with a protease inhibitor with a serine or metallo protease inhibitor, wherein said cell expresses or may be induced to express TMEM27.

In one embodiment, "treating" refers to both therapeutic treatment and prophylactic or preventative measures, wherein the object is to prevent or lessen the targeted pathologic condition or disorder as described hereinabove. Thus, in one embodiment, treating may include suppressing, inhibiting, preventing, treating, or a combination thereof. Thus, in one embodiment, "treating" refers inter alia to increasing time to sustained progression, expediting remission, inducing remission, augmenting remission, speeding recovery, increasing efficacy of or decreasing resistance to alternative therapeutics, or a combination thereof. In one embodiment, "suppressing" or "inhibiting", refers inter alia to delaying the onset of symptoms, preventing relapse to a disease, decreasing the number or frequency of relapse episodes, increasing latency between symptomatic episodes, reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

In one embodiment, symptoms are primary, while in another embodiment, symptoms are secondary. In one embodiment, "primary" refers to a symptom that is a direct result of diabetes, while in one embodiment, "secondary" refers to a symptom that is derived from or consequent to a primary cause. In one embodiment, the compounds for use in the present invention treat primary or secondary symptoms or secondary complications related to diabetes.

In another embodiment, "symptoms" may be any manifestation of a disease or pathological condition, which in one embodiment is diabetes, comprising Frequent urination, Excessive thirst, Extreme hunger, Unusual weight loss, Increased fatigue, Irritability, Blurry vision, low insulin levels, high blood or urinary glucose levels or a combination thereof.

In one embodiment, the term "diabetes" refers to a disease of a mammalian subject, with primary, or in another embodiment, secondary diabetes, or in another embodiment, type 1 NIDDM-transient, or in another embodiment, type 1 IDDM, or in another embodiment, type 2 IDDM-transient, or in another embodiment, type 2 NIDDM, or in another embodiment, type 2 MODY, which may manifest, as described, in Harrison's Internal Medicine, 14th ed. 1998.

According to this aspect of the invention, and in one embodiment, the subject is insulin resistant or, in another embodiment, hypoinsulinemic. In another embodiment, the subject is predisposed to diabetes. In another embodiment, the subject has maturity onset diabetes of the young (MODY).

In another embodiment, the methods of this invention may further comprise the step of administering to the subject an additional diabetes medication, as part of a combination therapy. In one embodiment, the diabetes medication may comprise a sulfonylurea, leptin, meglitinide, biguanide, thiazolidinedione, alpha-glucosidase inhibitor, or a combination thereof.

In another embodiment, the methods of this invention may further comprise administering to the subject, or in another embodiment, contacting cells in the subject, with a GLP-1 receptor agonist. In one embodiment, the GLP-1 agonist may include naturally occurring peptides such as GLP-1, exendin-3, and exendin-4 (see, e.g., U.S. Pat. No. 5,424,286; U.S. Pat. No. 5,705,483, U.S. Pat. No. 5,977,071; U.S. Pat. No. 5,670,360; U.S. Pat. No. 5,614,492), GLP-1 analogs or variants (see, for example, U.S. Pat. No. 5,545,618 and U.S. Pat. No. 5,981,488), and small molecule analogs. GLP-1 receptor agonists may be tested for activity as described in U.S. Pat. No. 5,981,488.

In another embodiment, the methods of this invention may further comprise administering PDX-1, or PYY, or a nucleic acid encoding the same.

It is to be understood that any embodiments described herein, with regards to a cell, composition, polypeptide, nucleic acid or vector of this invention, may be used in a method of this invention, and is to be considered an embodiment of this invention.

In one embodiment, this invention provides a method of assessing pancreatic islet mass in a subject, the method comprising the step of determining a concentration of TMEM27 in the serum of said subject and comparing said concentration versus that of a control.

In another embodiment, this invention provides a diagnostic tool and/or method for assessing hyperinsulinemia, hypoinsulinemia, diabetes or response to therapies for the treatment of the aforementioned conditions. In one embodiment, TMEM27 serves as a biomarker, whose concentration in a given body fluid, such as, for example, in serum, plasma or urine is a function of hyperinsulinemia, hypoinsulinemia or frank diabetes. In one embodiment, the secreted form of TMEM27 is a biomarker. According to this aspect of the invention, and in one embodiment, a method of diagnosis may be to determine levels of the TMEM27 protein, or fragments, secreted forms thereof, via standard assays, such as, for example, an ELISA assay. In one embodiment, disease severity is diagnosed as a function of the expression of the polypeptide. In another embodiment, changes in expression of the polypeptide, either at the protein or RNA level, may be a function of, in another embodiment, response to treatment, such as the treatment methods provided herein. In another embodiment, relative changes in expression may be assessed as a function of delivery of a nucleic acid or vector of this invention, which may represent a therapy of this invention. In one embodiment, the methods of this invention may employ the use of an antibody or antibody fragment, or composition comprising the same, of this invention. A kit comprising the antibody, nucleic acids, vector, cell and/or compositions of this invention are also encompassed by this invention.

In another embodiment, this invention provides a method for identifying proteins, which interact with a TMEM27 protein, or a secreted form thereof, the method comprising the step of contacting a TMEM27 polypeptide with a molecule of interest for a time and under conditions sufficient to facilitate specific interaction between said polypeptide and said molecule of interest and identifying said molecule of interest.

In one embodiment, TMEM27 may function as a hormone, and an interacting protein may comprise an unidentified receptor which is responsive to the hormone. In one embodiment, the interacting protein may comprise a known receptor involved in a metabolic pathway in a subject.

In one embodiment, such screening methods are well known in the art, and may comprise direct or indirect assessment of interactions between the two, such as, for example, analysis under denaturing versus non-denaturing conditions for gel electrophorcsis, use of chemical crosslinking agents, or other molecular means, such as, for example, use of the yeast 2 hybrid system.

It is to be understood that any means of determination of interacting partners with a TMEM27 protein of this invention may be accomplished and considered as a part of this invention, and is meant to include identification of the interacting partner, as well as a role for such a partner in the conditions described herein, for example, positive metabolic effects, such as their effect on enhancing pancreatic β-cell mass, or in another embodiment, deleterious metabolic effects, for example, such as the development of hyperinsulinemia.

The following are meant to provide materials, methods, and examples for illustrative purposes as a means of practicing/executing the present invention, and are not intended to be limiting.

### EXAMPLES

### Materials and Methods

### Animals

All animal models were housed in Laboratory of Animal Research Center (LARC), a pathogen-free animal facility at the Rockefeller University. The animals were maintained on a 12 hours light/dark cycle and fed a standard rodent chow. Genotyping of mutant mice was performed on DNA isolated from 3 weeks old mice by PCR.

### Antibodies

Two peptides: anti-Col-3: VQSAIRKNRNRINSAFFLD (SEQ ID NO: 1) and anti-Col-4: GIPCDPLDMKGGHINDGFLT (SEQ ID NO: 2) were synthesized and processed to >90% purity, conjugated to KLH and used for immunization of rabbits (Bethyl Laboratories, Texas). Antisera were affinity purified and tested by western blotting and immunohistochemistry. Affinity purified antisera were used for all studies. Other antibodies used for immunoblotting and immunohistochemistry were obtained from the following sources: anti-insulin (Linco), anti-glucagon (Linco), anti-V5 (Invitrogen), Rhodamine red conjugated donkey anti-guinea pig (Jackson Labs), Alexa 488 donkey anti-rabbit (Molecular Probes).

### Cell culture

MIN6 cells were cultured with DMEM medium containing 25 mM glucose, 15% fetal bovine serum, and 5.5 µM 2-mercaptoethanol. INS-1 cells were cultured with RPMI 1640 medium containing 25 mM glucose and 5% fetal bovine serum and 10 mM Hepes pH7.4. HepG2 cells were cultured with DMEM medium containing 25 mM glucose and 10% fetal bovine serum.

### Transient transfections and luciferase assay

Fugene reagent (Roche) for HepG2 cells and Lipofectamine 2000 (Invitrogen) for MIN6 cells were used according to the manufacturer's directions in transient transfections. 0.5 µg of luciferase reporter construct, expression vector and CMV-LacZ were added per 35 mm dish. Luciferase was normalized for transfection efficiency by the corresponding β-galactosidase activity [AlamJ., Cook, J.L.: Reporter genes: Application to the study of mammalian gene transcription. Anal. Biochem. 188:245-254, 1990].

For the luciferase construct, an 815 bp promoter region was cloned upstream of a luciferase promoter and coexpressed with a Tcf1 expression vector. The TMEM27 promoter sequence (mouse) was as follows:

atgtcattgcacccaatgactttaacgctcagtccttggataccccacatgtgagacggttgtaaccactgtaaggatttgagaaataatcagggagcctagc
tcacagtaagtagttcttagtcattgttaaatagttgcgctgaacaatgttgtcactagcactgtgtagcaacttaactaaatgacaggttcttagcagttctagc
cccttttaaagggttagcattcatttcttcctagtggcaccaggacagagcactgttcagggaaggtatcaccatcaccatcaccatcaccatcgtcatcacc
atcaccatcgtcatcacccaatgatcatcatctccagcgtgaggctttatatcattaatgttgagttttagacagtagccagatcttagagtttaacctagccatc
ctctagttgggaattgttaaccaaccaacagtctaggaagtcttgttccctttactctccagaggtgaaaaaggagttatatttgtttcctaagtatggtagcctg
tctttgaatcagattggttagcctgaaagggcacaaagattatgccaaagaaaactccatgctctccacgtttagcttaaccaaacaactacaggaggcagg
tgggaggcttctgattgtgtcagctcaggaaacattcttatgaggtagagtttgaggaagaacccactaacctgcttctcaaatagattttcgtaaataactga
caggggatggttcgcttctatggacgtattaacccaactgatgggcgttaattattaaacctttttagatggtggctcgctgattt (SEQ ID NO: 17).

In addition, the TMEM27 promoter was selectively mutated for Tcf1 sites, called the M1 and M2 promoter sequences, respectively, as follows:

Sequence of TMEM27-M1promoter (mouse):

atgtcattgcacccaatgactttaacgctcagtccttggataccccacatgtgagacggttgtaaccactgtaaggatttgagaaataatcagggagcctagc

tcacagtaagtagttcttagtcattgttaaatagttgcgctgaacaatgttgtcactagcactgtgtagcaacttaactaaatgacaggttcttagcagttctagc
cccttttaaagggttagcattcatttcttcctagtggcaccaggacagagcactgttcagggaaggtatcaccatcaccatcaccatcaccatcgtcatcacc
atcaccatcgtcatcacccaatgatcatcatctccagcgtgaggctttatatcattaatgttgagttttagacagtagccagatcttagagtttaacctagccatc
ctctagttgggaattgttaaccaaccaacagtctaggaagtcttgttccctttactctccagaggtgaaaaaggagttatatttgtttcctaagtatggtagcctg
tctttgaatcagattggttagcctgaaagggcacaaagattatgccaaagaaaactccatgctctccacgtttagcttaaccaaacaactacaggaggcagg
tgggaggcttctgattgtgtcagctcaggaaacattcttatgaggtagagtttgaggaagaacccactaacctgcttctcaaatagattttcgtaaataacact
gaggggatggttcgcttctatggacgtattaacccaactgatgggcgttaattattaaacctttttagatggtggctcgctgattt (SEQ ID NO: 18)

Sequence of TMEM27-M2 promoter (mouse):

atgtcattgcacccaatgactttaacgctcagtccttggataccccacatgtgagacggttgtaaccactgtaaggatttgagaaataatcagggagcctagc
tcacagtaagtagttcttagtcattgttaaatagttgcgctgaacaatgttgtcactagcactgtgtagcaacttaactaaatgacaggttcttagcagttctagc
cccttttaaagggttagcattcatttcttcctagtggcaccaggacagagcactgttcagggaaggtatcaccatcaccatcaccatcaccatcgtcatcacc
atcaccatcgtcatcacccaatgatcatcatctccagcgtgaggctttatatcattaatgttgagttttagacagtagccagatcttagagtttaacctagccatc
ctctagttgggaattgttaaccaaccaacagtctaggaagtcttgttccctttactctccagaggtgaaaaaggagttatatttgtttcctaagtatggtagcctg
tctttgaatcagattggttagcctgaaagggcacaaagattatgccaaagaaaactccatgctctccacgtttagcttaaccaaacaactacaggaggcagg
tgggaggcttctgattgtgtcagctcaggaaacattcttatgaggtagagtttgaggaagaacccactaacctgcttctcaaatagattttcgtaaataactga
caggggatggttcgcttctatggacgtattaacccaactgatgggccaatattattaaacctttagatggtggctcgctgattt (SEQ ID NO: 19).

### Crosslinking of proteins in cell lysates

MIN6 cells, grown to 90 % confluency in 6 well plates, were harvested and resuspended in reaction buffer and reagent in 50 µl volume and incubated for 2 hrs at 4°C. Reactions were stopped by adding 50 mM Tris pH 7.4 for 10 min on ice. Cells were then washed with PBS and lysed in RIPA buffer in the presence of protease inhibitors for 15 min at 4°C. Cell lysates were centrifuged for 5 min at 10,000 x g and the supernatants were subjected to reducing and non-reducing SDS-PAGE followed by immunoblotting. Crosslinking reagents and buffers: BMH (Pierce) was dissolved in DMSO and incubated in PBS, and DTBP (Pierce) was dissolved in water and incubated in 0.2 M triethanolamine pH 8.0.

### Inhibition and enzymatic cleavage of N-glycosylation

MIN6 cells that express pV5-TMEM27 were incubated with indicated concentrations of tunicamycin (Sigma) dissolved in DMSO and with DMSO alone for 12 hours at 37°C. Cell lysates were then subjected to SDS-PAGE and immunoblotting with anti-V5 antibody.

Intact N-linked glycans were removed from secreted portion of TMEM27 with the recombinant enzyme N-glycanase (Prozyme, San Leandro, CA). Supernatants from MIN6 cells and pancreatic islets were denatured in 20 mM sodium phosphate pH 7.5, 0.1 % SDS and 50 mM β-mercaptoethanol by heating at 100°C for 5 min. NP-40 was added to a final concentration of 0.75 % and reaction was incubated with N-glycanase for 3 h at 37°C.

### Electrophoretic mobility shift assay (EMSA) and chromatin immunoprecipitation (ChIP)

EMSA analysis was performed with 10 µg of whole cell extracts in binding buffer (20 mM Hepes pH 7.9, 10 % glycerol, 150 mM NaCl, 1 mM DTT). Whole cell extracts were incubated with ³²P-labeled ds-stranded oligonucleotide probes containing the wildtype or mutant HNF-1 binding sites in the TMEM27 promoter (sequences: 5'-GGAGATTTTCGTAAATAACTGACA -3' (SEQ ID NO: 3), 5'-GGGCGTTAATTATTAAACCTTTTA-3' (SEQ ID NO: 4) and 5'-GGGCAGAGATTATTAAACCTTTTA-3' (SEQ ID NO: 5), respectively). Supershifts were carried out with an anti-Tcf1 antibody (Geneka Biotechnology Inc., Montreal, Canada). ChIP analysis was carried out using isolated primary hepatocytes from *C57*/*B6* mice or MIN6 cells, and the ChIP Assay kit (Upstate Cell Signaling Solutions, Lake Placid, NY) according to the manufacturer's protocol. Tcf1 was precipitated with anti-HNF-1α antibody, and DNA was amplified using primers x and y (sequences: 5'-ACAGGAGGCAGGTGGGAGGCTTCT -3' (SEQ ID NO: 6) and 5'-CCCGGATTAGGGTATCGGAGAA -3') (SEQ ID NO: 7). Primers for apoM were 5'-GGGCTCAGCTTTCCTCCTA-3' (SEQ ID NO: 8) and 5'-CTCCGCCTTAACTGTTCTCTGATG -3') (SEQ ID NO: 9).

### Whole Cell extract preparation

MIN6 cells were grown to 90% confluency in 150 mm tissue culture dishes. Cells were washed once in ice-cold PBS and scraped into 3 ml PBS. Cells were centrifuged at 4,000 x g for 4 min and resuspended in 2 volumes of high-salt extraction buffer (400 mM KCl, 20 mM Tris pH 7.5, 20 % glycerol, 2 mM DTT, 1x complete TM protease inhibitors (Boehringer Mannheim), and 20 µg/mL Aprotinin). Cell lysis was performed by freezing and thawing, and the cellular debris was removed by centrifugation at 16,000 x g for 10 min at 4°C.

### Hormone measurements

Insulin and glucagon were extracted from pancreata with acid ethanol (10% glacial acetic acid in absolute ethanol), sonicated for 10 min, and centrifuged 2 times at 4°C at 12,000xg for 10 min. Supernatants were collected and stored at -20°C for insulin determination by using an sensitive insulin or glucagon RIA kit (Linco Research).

### Pancreatic islet and RNA isolation

Pancreatic islets were isolated from 6 to 8 week-old mice. We used collagenase digestion and differential centrifugation through Ficoll gradients. Total RNA was then extracted using TRIzol reagent (Gibco-BRL) and following the manufacturer's instructions. Contaminating genomic DNA was removed using 1µl of RNase free DNase-I (Boehringer) per 5µg of RNA.

### Islet morphometry

The pancreata were fixed in paraformaldehyde and stained for insulin and glucagon as described above. Sections (7µm) through the entire pancreas were taken, and every sixth section was used for morphometric analysis. At least 288 non-overlapping images (pixel size 0.88µm) were scanned using a confocal laser-scanning microscope (Zeiss LSM 510, Germany). The parameters measured in this study were analyzed using integrated morphometry analysis tool in the Metamorph Software Package (Universal Imaging Corporation, PA). The area covered by cells stained by insulin or glucagon was integrated using stained objects that are greater than 3 pixel in size.

### RT-PCR

Total RNA was extracted using TRIZOL reagent (Invitrogen) and 10 µg of RNA was treated with 5U of RNase-free DNase-I (Ambion). cDNA was synthesized using Moloney leukemia virus reverse transcriptase with dNTPs and random hexamer primers (Invitrogen). The cDNAs provided templates for PCRs using specific primers in the presence of [α-³²P]dCTP and Taq polymerase as previously described [Shih DQ, Screenan S, Munoz KN, Philipson L, Pontoglio M, Yaniv M, Polonsky KS, Stoffel M. (2001) Loss of HNF-1alpha function in mice leads to abnormal expression of genes involved in pancreatic islet development and metabolism. Diabetes 50:2472-80].

### Immunoblotting and Immunohistochemistry

Cytosolic protein extracts were separated by SDS-PAGE (4-15%) and transferred onto a nitrocellulose membrane (Schleicher & Schuell) by electroblotting. TMEM27 was detected with anti-Col3 and anti-Col4 antisera (1:500). Membranes were incubated with primary antibodies overnight at 4°C. Incubations containing the secondary antibody were performed at RT for 1 hr. Non-reducing SDS-PAGE was performed by omitting DTT from sample buffer.

MIN6 cells were plated on coated slides (Nalge Nunc Int.) and fixed with 4% paraformaldehyde at 4°C for 20 min. Slides were incubated in 0.01% saponin with 3% normal donkey serum in PBS for 30 min at room temperature and anti-Col3 and anti-Col4 (1:20) were added overnight at 4°C. Secondary antibody was added for 30 min at RT.

Pancreata were fixed in 4 % paraformaldehyde at 4°C for 4 hours and embedded in paraffin. Seven µm sections were cut and after deparaffinization, antigen-unmasking was performed by microwaving slides in 0.01 M sodium citrate pH 6.0. Sections were permeabilized in 0.1 % Triton-X-100 and incubated in 3 % normal donkey serum in PBS for 30 min. Staining was completed as above.

### Electron microscopy studies

Min 6 cells were fixed in 4% paraformaldehyde and 0.02% glutaraldehyde in 0.1M cacodylate buffer, pH 7.4 for 2 hours. The cells were washed with PBS and embedded in 10% gelatin and refixed as above. Cell pellets were cryo-protected using a 2.3M sucrose solution in PBS, and samples were stored in liquid nitrogen until use (Tokuyasu, K. T. 1973. J. Cell. Biol. 57-551-565). Cryo-ultrathin sections were cut using glass knives in a Reichert-Jung FC-4E cryoultramicrotome. The sections were collected on Formvar-carbon coated nickel grids, blocked with 1% BSA-PBS and incubated with anti-col4 at a 1:10 dilution. Incubation was stopped by washing 2 times with PBS, 15 min each. The sections were then incubated with goat anti-rabbit IgG conjugated to 10 nm gold particles (Amersham Life Science, Arlington Heights, IL). The grids were processed and stained according to Griffiths et al. 1983. Methods Enzymol. 96:466-485).

### Thymidine incorporation studies

[³H]Thymidine incorporation in 5 x 10⁴ cells/well in 24-well culture plates was assayed as follows. 48 hours after electroporation, cells were incubated in growth arrest medium (0.5% FCS) for the next 24 hours and then incubated for an additional 24 hours in normal growth medium. For the last 4 h of the incubation, 0.25 µCi/well [³H]methylthymidine (Perkin Elmer) was added. After completion, cells were rinsed twice in ice-cold PBS, and incubated with 10% trichloroacetic acid (TCA) on ice for 20 min. After washing with 10% TCA, cells were solubilized in 0.2M NaOH/1% SDS for 10 min at room temperature. TCA-insoluble materials were neutralized with 0.2M HCl, and radioactivity was determined by a liquid scintillation counter.

### RNA interference

Synthetic siRNAs were synthesized by Dharmacon Research (Lafayette, CO). siRNAs were designed for mouse TMEM27 (NM 020626), PC1/3 (NM 013628), PC2 (NM_008792), furin (NM 011046), and carboxypeptidase E (NM_013494) sequences. 3µg of each siRNA per 1 x 10⁶ cells were electroporated into MIN6 cells.

### Statistical analysis

Results are given as mean ± SD. Statistical analyses were performed by using a Student's *t*-test, and the null hypothesis was rejected at the 0.05 level.

### EXAMPLE 1

### Tcf1 Regulates Expression of a Novel Protein in Pancreatic β-cells

In an attempt to identify mitogenic factors in pancreatic β-cells, gene expression in isolated pancreatic islets of *Tcf1*^{-/-} wildtype littermates was compared using Affymetrix™ oligonucleotide expression arrays. This analysis identified a gene encoding a transmembrane-spanning protein (TMEM27) that showed a 16-fold decrease in expression levels of *Tcf1*^{*-*/*-*} mice compared to controls. This result was confirmed by RT-PCR in an independent group of animals (Figure 1a). To study whether Tcf1 is a direct activator of TMEM27 transcription, the TMEM27 promoter was analyzed and two conserved high-affinity binding sites were identified in its regulatory region (Figure 1b). The upstream regulatory region in humans (SEQ ID NO: 10) and in mice (SEQ ID NO: 11) comprised Tcf binding sites, beginning at about -117 to about -102, and -62 to about -47 in humans (SEQ ID NO: 12 and SEQ ID NO: 13) and mice (SEQ ID NO: 14 and SEQ ID NO: 15), respectively.

An 815 bp promoter region (SEQ ID NO: 17) was cloned upstream of a luciferase promoter and coexpressed with a Tcf1 expression vector. Transient co-transfection of Tcf1 and the TMEM27 promoter led to a >5-fold activation of luciferase activity (Figure 1c). When each Tcf1 site in the TMEM27 promoter was selectively mutated, transcriptional activation was reduced 70-80% (Figure 1c). Electrophoretic gel mobility shift assays (EMSA) using double stranded ³²P-labeled oligonucleotides containing the Tcf binding sites and MIN6 cell extracts detected DNA/protein complexes (Figure 1d). The specificity of protein binding to these sites was determined by competition assay using unlabeled oligonucleotides and by further shifting the DNA-Tcf1 complex using specific anti-Tcf1 antiserum (Figure 1d). In order to determine whether Tcf1 increases expression of TMEM27 directly chromatin immunoprecipitation (ChIP) was performed. Tcf1 bound to binding sites in the TMEM27 promoter of MIN6 cells, but not in hepatocytes where TMEM27 was not expressed (Figure 1e). These data confirm the functionality of the Tcf1 binding sites *in vivo* and demonstrated that Tcf1 was required for expression of TMEM27 in pancreatic β-cells.

### EXAMPLE 2:

### TMEM27 Expression is Regulated During Pancreas Development

Immunohistochemical analysis indicated the pattern of expression of TMEM27 during mouse pancreatic development. In newborn and adult pancreas, TMEM27 expression was restricted to β-cells of the islet. During development of the pancreas, TMEM27 was expressed at the earliest time when hormone positive (mainly glucagon-positive cells) are apparent. At embryonic days E11.5 and E12.5, TMEM27 expression was localized in cells that express glucagon and insulin. During the peak period of endocrine cell expansion, from embryonic day 13.5 to 18.5, TMEM27 mainly co-localized with glucagon until right before birth at E18.5 when it was also detected in insulin positive cells. In newborn and adult pancreas, TMEM27 was no longer detected in α-cells of the islets and expression was confined to β-cells (Figure 2).

### EXAMPLE 3:

### TMEM27 is an N-linked Glycoprotein and Forms Dimers In Vivo

TMEM27 contains two predicted N-glycosylation sites at amino acid residues 76 and 93. MIN6 cells were treated with tunicamycin, which inhibits N-glycosylation. Cells were incubated in the presence of increasing concentrations of tunicamycin, and protein extracts were prepared and analyzed by SDS-PAGE and immunoblotting. Two bands with increased electrophoretic mobility appeared upon treatment with tunicamycin, whereas the high molecular weight protein disappeared (Figure 3a). This result was confirmed with an *in vitro* assay using N-glycanase, an enzyme that releases intact N-linked glycans. Western blot analysis of MIN6 cell extracts that were incubated with this enzyme showed similar results to tunicamycin treatment (Figure 3b).

Chemical cross-linking experiments testing whether TMEM27 protein exists as a multimer were conducted. MIN6 cell extracts were incubated in the presence of two different cross-linking reagents; BMH (bismaleimidohexane), a membrane-permeable, non-cleavable compound and DTBP (dimethyl 3, 3'-dithiobispropionimidate), a membrane permeable, cleavable compound prior to SDS-PAGE analysis that was performed under reducing and non-reducing conditions. Non-reducing western blots revealed a band of exactly twice the molecular weight than the TMEM protein in western blots under reducing conditions. Cell extracts treated with BMH showed the dimer protein in both reducing and non-reducing blots. However, the protein dimers disappeared under reducing conditions in the presence of DTBP (Figure 3b). The results were similar in MIN6 cells that endogenously express TMEM27 and in N2A cells that were transiently transfected with TMEM27 expression vector (data not shown).

Taken together, these data indicated that the TMEM27 protein is N-glycosylated, and exists as a dimer.

### EXAMPLE 4:

### TMEM27 is Processed in and Secreted from Pancreatic β-Cells

TMEM27 was predicted to be a transmembrane protein with an N-terminal extracellular domain. Two antibodies (α-Col-3 and α-Col-4) that recognize peptides from extra- and intracellular domains of the protein, respectively, were generated. In Western blotting experiments, α-Col-4 detected two bands in whole cell lysates of MIN6 cells (Figure 4a). The higher molecular weight band corresponded to the predicted molecular weight of the glycosylated full-length protein. The lower band (≈25 kD) was also specific since it could be detected by Western blotting in cell lysates following transfection with a vector (p-TMEM27.V5-His) expressing a C-terminal V5-Tag fusion protein and an anti-V5 antibody (Figure 4b).

In order to demonstrate whether the 25 kD band represented a C-terminal part of a cleaved form of TMEM27 and/or whether it was secreted, supernatants from different cell lines (including neuronal N2A cells, HepG2c ells, Hek293 and M-1 cells that were derived from collecting ducts of the kidney and which exhibit endogenous expression of TMEM27) were probed with the α-Col-3 antibody. Cells were cultured for 48 hours in serum free medium, and probed by Western blotting using α-Col-3, with a band corresponding to a ≈25kDa protein detected in the clonal β-cell lines MIN6 and INS1E (Figure 4c, data not shown). This putative cleaved and secreted protein could also be detected in media incubated with isolated mouse pancreatic islets (Figure 4d). Interestingly, in spite of robust expression of full-length protein in cell-lysates, it was not possible to detect this band in Western blot analysis in the culture medium of non-β-cell lines that were transiently transfected with a TMEM27 expression vector (pTMEM27).

In contrast, overexpression of TMEM27 in MIN6 and INS1E cells led to increased amounts of secreted protein in their respective culture media. siRNA-mediated reduction in TMEM27 protein levels correlated with decreased levels of the secreted form of TMEM27 in MIN6 cell supernatants (Figure 4e). Lastly, in order to irrefutably prove that N-terminal portion of TMEM27 is cleaved and secreted from β-cells, an expression vector (pHA-TMEM27) was generated in which a nine amino acid HA-epitope tag was inserted between amino acid residues 39 and 40 of the TMEM27 protein, and MIN6 cells transfected with this vector secreted a protein with similar size in the supernatants that could be detected with an anti-HA antibody. This protein was not detected in control cells or cells expressing the untagged protein (Figure 4f). Together, these data demonstrate that TMEM27 is a pancreatic β-cell-specific, cleaved and secreted transmembrane protein.

### EXAMPLE 5:

### TMEM27 is Localized to Insulin Secretory Granules and the Plasma Membrane

Immunofluorescence studies using anti-Col-3 and -4 antibodies demonstrated TMEM27 localization in pancreatic β-cells. MIN6 cells were grown on slides, fixed, permeabilized and treated with primary antibodies. Specific staining was detected on the plasma membrane as well as in the perinuclear compartment and in granules (Figure 5a). This result is consistent with TMEM27 being localized in insulin secretory granules and with its being transported to the plasma membrane via these vesicles. Electron microscopy studies using immuno-gold labeled anti-Col-4 antibody demonstrated TMEM27 localization within insulin secretory granules (Figure 5b). Nanogold particles were associated with the plasma membrane in proximity to vesicle /membrane fusion events (Figure 5c), indicating TMEM27 is a plasma membrane protein which co-localized with insulin secreting granules.

### EXAMPLE 6:

### Increased Expression of TMEM27 in Ob/Ob Islets and Enhanced B-Cell Replication In Vitro

TMEM27 expression in hypertrophied islets of *ob*/*ob* and aP2-Srebp-1c transgenic mice was evaluated to determine relative expression, as compared to controls. Pancreatic islets from 6-8 week old mice were isolated and gene expression levels were compared to their wildtype littermates. A 2.1- and 1.7-fold increase in TMEM27 levels of *ob*/*ob* and aP2-Srebp-1c islets, respectively, was found in semiquantitative RT-PCR assays, in comparison to control values (Figures 6a and 6b). In addition, islets from *ob*/*ob* and *aP2-Srebp-1c* mice exhibited a significant increase in secretion of cleaved TMEM27 into the medium compared to size-matched islet of wildtype littermates (Figure 6c, data not shown). To determine whether TMEM27 stimulates pancreatic β-cell replication, the effect of TMEM27 expression on the proliferation of MIN6 cells was assessed. Cells were electroporated with either plasmid pTMEM27 or siRNAs targeting TMEM27. Cell replication was assessed by cell counting and measuring incorporation of [³H]thymidine 48 h after electroporation (Figure 6d-g). MIN6 cells that were transfected with pTMEM27 exhibited ≈5-fold overexpression compared to pcDNA3 transfected cells and showed ≈3-fold increase in [³H]thymidine incorporation and increased cell density 48 hours after plating (Figure 6e, g). In contrast, MIN6 cells that had reduced expression of protein following siRNA treatment showed significantly lower [³H]Thymidine incorporation and cell density (Figure 6d, f). Furthermore, no changes in apoptosis in cells with increased or reduced expression of TMEM27 was observed (data not shown), indicating expression does not affect cell death. These results indicate that TMEM27 regulates cell proliferation in MIN6 cells.

### EXAMPLE 7:

### Trangenic Mice Expressing TMEM27 in Pancreatic Islets Exhibit Islet Hypertrophy

Pancreatic β-cell-specific transgenic mice were generated via pronuclear microinjection of a vector construct in which the murine TMEM27 cDNA was under the control of the rat insulin promoter. RT-PCR and immunoblot analysis showed that TMEM27 expression was ≈4-fold increased in transgenic mice compared to wildtype littermates (Figure 7A). Islet mass was studied in 8-12 week-old transgenic animals by pancreatic islet morphometry. Transgenic mice exhibited normal islet morphology (based on immunohistochemistry using insulin/glucagon costaining) but had an approximately 2-fold increase in islet mass compared to wildtype littermates (Figures 7B and 7C). The increase in islet mass was further validated by measuring the total insulin content of transgenic and wildtype mice. The total insulin content was also significantly increased in transgenic mice overexpressing TMEM27 (Figure 7D). Fasting and postprandial plasma glucose and insulin levels were similar in transgenic and control mice, indicating that pancreatic β-cells in transgenic mice had no major abnormalities in glucose sensing (data not shown). Together, these data demonstrate that TMEM27 regulates islet growth in vivo.

### EXAMPLE 8:

### BB94 inhibits shedding of TMEM27

The pancreatic beta-cell line MIN6 was cultured with DMEM medium containing 25 mM glucose, 15% fetal bovine serum, and 5.5 µM 2-mercaptoethanol. MIN6 cells were plated at 50% confluency in 12 well plates and incubated for 24 hrs. Cells were washed with OPTI-MEM (Invitrogen) and incubated with either DMSO or protease inhibitor BB94 (1 µM) for 15 minutes in OPTI-MEM at 37°C. After the above incubation, cells were incubated with DMSO, PMA (Sigma P8139), BB94, or PMA + BB94 for 2 hrs in OPTI-MEM. 30 µl of each supernatant was collected for SDS-PAGE. Immunoblots were performed using anti-TMEM27 antibodies recognizing the N-terminus (cleaved portion) of TMEM27 (Figure 8). Immunoblots using anti-V5 antibody, which recognizes the C-terminal (intracellular) portion of TMEM27, was used as a control, showing that PMA and BB94 do not change the expression levels of TMEM27 (Figure 8). BB94, but not PMA, decreases expression levels of the N-terminus of TMEM27 (Figure 8) without changing the expression levels of the C-terminus of TMEM27. Since BB94 is a known inhibitor of serine proteases and metalloproteases, this may indicate that TMEM27 is a substrate of a protease belonging to serine protease or metalloprotease family.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. An isolated nucleic acid comprising a regulatory region of a gene encoding a TMEM27 protein, wherein said regulatory region comprises at least one high-affinity binding site for a Tcf1 protein.

2. The isolated nucleic acid of claim 1, wherein said regulatory region has a nucleic acid sequence corresponding to or homologous to SEQ ID NO: 10 or 11.

3. The isolated nucleic acid of claim 1, wherein said binding site comprises a nucleic acid sequence corresponding to or homologous to SEQ ID NO: 12, 13, 14, or 15.

4. A cell comprising the nucleic acid of claim 1.

5. The cell of claim 4, wherein said cell is a pancreatic β cell.

6. A vector comprising the nucleic acid of claim 1.

7. An isolated polypeptide, comprising a secreted form of a TMEM27 protein, wherein said polypeptide is about 25 kDa in size, and comprises an N-terminal fragment of a sequence corresponding to, or homologous to SEQ ID NO: 16.

8. A method of increasing pancreatic β-cell mass, said method comprising contacting a pancreatic β-cell or a cell which may be induced to become a pancreatic β-cell with a TMEM27 polypeptide and providing conditions favorable for β-cell proliferation.

9. The method of claim 8, wherein said cell is contacted in vitro or ex vivo.

10. The method of claim 8, wherein said cell is contacted in vivo.

11. The method of claim 8, wherein said cell is a stem or progenitor cell.

12. The method of claim 8, wherein said cell is isolated from a subject suffering from or predisposed to diabetes.

13. The method of claim 12, further comprising the step of administering said cell to said subject.

14. The method of claim 13, wherein said cell is autologous or allogeneic with respect to said subject.

15. The method of claim 8, wherein said TMEM27 polypeptide comprises a full-length protein, or a fragment thereof.

16. The method of claim 8, further comprising the step of contacting said cell with a protease inhibitor.

17. The method of claim 16, wherein said protease inhibitor is a serine protease inhibitor or a metalloprotease inhibitor.

18. The method of claim 17, wherein said protease inhibitor is BB94.
